(19) Europäisches Patentamt
European Patent Office
Office européen des brevets

(11) **EP 3 115 785 A2**

(12) **EUROPEAN PATENT APPLICATION**

(43) Date of publication:
**11.01.2017 Bulletin 2017/02**

(51) Int Cl.:
***G01N 33/68*** *(2006.01)*

(21) Application number: **16170575.1**

(22) Date of filing: **04.02.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **04.02.2008 US 63515 P**
**23.05.2008 US 55737 P**
**01.08.2008 US 85623 P**

(62) Document number(s) of the earlier application(s) in accordance with Art. 76 EPC:
**09707520.4 / 2 245 466**

(71) Applicant: **Banyan Biomarkers, Inc.**
**Alachua, FL 32615 (US)**

(72) Inventors:
• **WANG, Kevin**
**Gainsville, FL 32607 (US)**
• **HAYES, Ronald, L.**
**Alachua, FL 32615 (US)**

(74) Representative: **ABG Patentes, S.L.**
**Avenida de Burgos, 16D**
**Edificio Euromor**
**28036 Madrid (ES)**

Remarks:
•This application was filed on 20.5.2016 as a divisional application to the application mentioned under INID code 62.
•The references to the drawing (Fig.9) are deemed to be deleted (Rule 56(4) EPC).

(54) **PROCESS TO DIAGNOSE OR TREAT BRAIN INJURY**

(57) A process for diagnosing and treating a neurological condition in a subject is provided that includes assaying a biological sample of a subject for the presence of one or more biomarkers; diagnosing a neurological condition based on a ratio of one or more of the biomarkers in the sample; and administering a therapeutic to the subject to alter the ratio of one or more biomarkers. The diagnosis of numerous neurological conditions such as brain injury or multiple organ injury is provided.

**Description**

CROSS REFERENCE TO RELATED APPLICATIONS

This application claims priority to US Provisional Application No. 61/063,515 filed February 4, 2008; US Provisional Application No. 61/055,737 filed May 23, 2008; and US Provisional Application No. 61/085,623 filed August 1, 2008. The contents of each provisional application is incorporated herein by reference as if each were explicitly and fully expressed herein.

FIELD OF THE INVENTION

The present invention relates generally to process of diagnosis or treatment of injury. The inventive process may simultaneously diagnose and treat injury simultaneous diagnosis and treatment. Traumatic brain injury is diagnosed and treated alone or inclusive of multi-organ trauma in an individual.

BACKGROUND OF THE INVENTION

Traumatic brain injury (TBI) is the leading cause of death and disability in persons under 45 years of age in industrialized countries (McAllister, 1992). Of the 1.5 million head traumas estimated to occur each year in the United States, 500,000 are likely to require hospitalization, and 80,000 result in some form of chronic disability (Langlois et al., 2006). The Center for Disease Control (CDC) estimates that at least 5.3 million Americans, or about 2% of the population, currently have a long-term requirement for assistance with daily living activities as a result of TBI (Langlois et al., 2006). Furthermore, total health costs for TBI amount to roughly $35 billion annually (Max et al., 1991). Despite the prevalence and severity of this form of injury, no effective treatment has yet been developed.

Physicians commonly attempt to distinguish between mild traumatic brain injury (MTBI) and TBI. MTBI is commonly defined by clinical symptoms including "[a]ny period of observed or self-reported transient confusion, disorientation, or impaired consciousness; [a]ny period of observed or self-reported dysfunction of memory (amnesia) around the time of injury; [o]bserved signs of other neurological or neuropsychological dysfunction, such as- seizures acutely following head injury; [a]mong infants and very young children: irritability, lethargy, or vomiting following head injury; [s]ymptoms among older children and adults such as headache, dizziness, irritability, fatigue, or poor concentration, when identified soon after injury, can be used to support the diagnosis of mild TBI, but cannot be used to make the diagnosis in the absence of loss of consciousness or altered consciousness. Any period of observed or self-reported loss of consciousness lasting 30 minutes or less." (National Center for Injury Prevention and Control. *Report to Congress on Mild Traumatic Brain Injury in the Unites States: Steps to Prevent a Serious Public Health Problem.* Atlanta, GA: Centers for Disease Control and Prevention; 2003.) Symptoms traditionally encompassing TBI include unconsciousness for more than 30 minutes; post traumatic amnesia lasting more than 24 hours; and penetrating cranialcerebral injury. *Id*.

The field of clinical neurology remains frustrated by the recognition that secondary injury to a central nervous system tissue associated with physiologic response to the initial insult could be lessened if only the initial insult could be rapidly diagnosed or in the case of a progressive disorder before stress on central nervous system tissues reached a preselected threshold. Traumatic, ischemic, and neurotoxic chemical insult, along with generic disorders, all present the prospect of brain damage. While the diagnosis of severe forms of each of these causes of brain damage is straightforward through clinical response testing and computed tomography (CT) and magnetic resonance imaging (MRI) testing, these diagnostics have their limitations in that spectroscopic imaging is both costly and time consuming while clinical response testing of incapacitated individuals is of limited value and often precludes a nuanced diagnosis. Additionally, owing to the limitations of existing diagnostics, situations under which a subject experiences a stress to their neurological condition such that the subject often is unaware that damage has occurred or seek treatment as the subtle symptoms often quickly resolve. The lack of treatment of these mild to moderate challenges to neurologic condition of a subject can have a cumulative effect or subsequently result in a severe brain damage event which in either case has a poor clinical prognosis.

In order to overcome the limitations associated with spectroscopic and clinical response diagnosis of neurological condition, there is increasing attention on the use of biomarkers as internal indicators of change as to molecular or cellular level health condition of a subject. As detection of biomarkers uses a sample obtained from a subject and detects the biomarkers in that sample, typically cerebrospinal fluid, blood, or plasma, biomarker detection holds the prospect of inexpensive, rapid, and objective measurement of neurological condition. With the attainment of rapid and objective indicators of neurological condition allows one to determine severity of a non-normal brain condition on a scale with a degree of objectivity, predict outcome, guide therapy of the condition, as well as monitor subject responsiveness and recovery. Additionally, such information as obtained from numerous subjects allows one to gain a degree of insight into the mechanism of brain injury.

Biomarkers of central nervous system (CNS) injury could provide physicians and laboratory studies with quantifiable neurochemical markers to help determine not only the severity and cellular pathology of injury, but also provide a surrogate marker of therapeutic interventions. While a number of potential biochemical markers for TBI have been proposed (Pineda et al., 2007), several studies have focused on breakdown products of $\alpha$II-spectrin proteolysis as biomarkers of CNS injury in rodents (Pike et al., 2004; Ringger et al., 2004) and humans (Pineda et al., 2007). A number of biomarkers have been identified as being associated with severe traumatic brain injury as is often seen in vehicle collision and combat wounded subjects. These biomarkers have included spectrin breakdown products such as SBDP150, SBDP150i, SBDP145 (calpain mediated acute neural necrosis), SBDP120 (caspase mediated delayed neural apoptosis), UCH-L1 (neuronal cell body damage marker), and MAP-2 dendritic cell injury associated marker. The nature of these biomarkers is detailed in U.S. Patents 7,291,710 and 7,396,654, the contents of which are hereby incorporated by reference.

Without being restricted to one theory or model, one proposed delineation between MTBI and TBI are the recognizable increase or decrease in molecular biomarkers in biological fluids following injury. Illustrative examples of molecular markers include those described by Kobeissy FH, et al., Mol Cell Proteomics, 2006; 5:1887-1898. A proposed definition of TBI is the presence of at least one recognizable molecular biomarker with at least two-fold increased or decreased levels in cortical tissue 48 h following experimental TBI (rat controlled cortical impact with controlled cortical impact at a 1.6 mm depression depth, equivalent to severe TBI in humans).

Alpha-II-spectun breakdown products (SBDP) present a potential protein biomarker for TBI. Alpha-II-spectun is primarily enriched in brain and is localized in neurons rather than glia. Furthermore, Alpha-II-spectun appears to be localized in axons (Czogalla and Sikorski, 2005; Riederer et al., 1986). Alpha-II-spectrin is cleaved by two cysteine proteases: calpain and caspase. Calpain, which exists in a quiescent state in the resting cell, is induced to a hyperactive state in response to significant elevations in intracellular calcium, and accompanies TBI (Fineman et al., 1993). This enzyme cleaves Alpha-II-spectlin into 150 and 145 kDa fragments. Calpain proteolysis is primarily associated with necrotic oncosis (Kampfl et al., 1997; Liu et al., 2004; Wang, 2002). Caspase, the activation of which is associated with apoptotic cell death, cleaves spectrin into distinct 150 and 120 kDa fragments (Pike et al., 2001; Wang, 2000). This differential cleavage permits not only an indication of CNS-specific hyperactivation of spectrin cleavage enzymes in response to injury, but also an assessment of the relative significance of necrosis and/or apoptosis as contributory factors in the injury pathology. Thus, these SBDPs can be considered biomarkers for TBI (Wang et al., 2005).

Prior studies examining excitotoxic mechanisms of TBI pathology focused on the neurotransmitter glutamate. However, considerable evidence indicates acetylcholine-related toxicity to be likewise of great importance in CNS pathologies. Increases in acetylcholine (ACh) levels associated with brain trauma were documented over fifty years ago in laboratory as well as clinical settings (Bornstein, 1946; Sachs, 1957). Comparatively higher levels of the neurotransmitter were noted with increasing severity of the injury (Tower and McEachern, 1949). More recent studies confirmed elevated ACh in brain cerebrospinal fluid (CSF) following experimental TBI (Gorman et al., 1989; Lyeth et al., 1993a) and ischemia (Kumagae and Matsui, 1991), as well as the injurious nature of high levels of muscarinic cholinergic receptor activation through application of cholinomimetics (Olney et al., 1983; Turski et al., 1983). Several studies also demonstrated that acute administration of muscarinic antagonists improves behavioral recovery following experimental TBI (Lyeth et al., 1988a; Lyeth et al., 1988b; Lyeth and Hayes, 1992; Lyeth et al., 1993b; Robinson et al., 1990).

While several potential markers have been proposed, no definitive marker or process has been shown capable of diagnosing and distinguishing between MTBI and TBI or of demonstrating successful or therapeutic advantage of therapeutic administration. This shortcoming is further apparent should an individual also suffer from multiple organ injury. Further, brain injuries are commonly difficult to treat effectively, and successful outcome commonly depends on how rapidly an individual is diagnosed with a particular injury subtype. Thus a need exists for a sensitive and specific biochemical marker(s) of TBI with the diagnostic ability to evaluate postconcussion intracranial pathology to improve patient management and facilitate therapeutic evaluation.

SUMMARY OF THE INVENTION

An inventive process for diagnosing and treating a neurological condition in a subject is provided that includes assaying a biological sample of a subject for the presence of one or more biomarkers; diagnosing a neurological condition based on a ratio of one or more of the biomarkers in the sample; and administering a therapeutic to the subject to alter the ratio of one or more biomarkers. The invention diagnoses numerous neurological conditions such as brain injury, or multiple organ injury. Preferably, the inventive process is useful in diagnosing percussive brain injury, ischemic stroke, and multiple-organ injury.

One or more biomarkers are used in the invention illustratively including $\alpha$-II spectrin; a spectrin breakdown product; MAP2; neuronal degeneration; ubiquitin carboxyl-terminal esterase; a ubiquitin carboxyl-terminal hydrolase; a neuronally-localized intracellular protein; MAP-tau; C-tau; Poly (ADP-ubose) polymerase (PARP); a collapsin response mediator protein; breakdown products thereof, derivatives thereof, and combinations thereof. In a preferred embodiment

3

a biomarker is a ubiquitin carboxyl-terminal hydrolase, a spectrin breakdown product, or MAP2.

The inventive method detects a ratio of a biomarker relative to a known or determined baseline level of the same biomarker or that of a different biomarker. Severe injury is distinguished from a mild injury in that a severe injury demonstrates a ratio of 2 or greater of biomarker relative to baseline level and a mild injury demonstrates less than a two-fold ratio of biomarker relative to baseline level. Optionally, the severity of injury is distinguished if the ratio of biomarker to baseline level is less than 0.5.

Numerous therapeutics are operable herein. Preferably, a therapeutic is a muscarinic receptor agonist. Illustrative examples of therapeutics include: dicyclomine, scoplamine, milameline, N-methyl-4-piperidinylbenzilate NMP, pilocarpine, pirenzepine, acetylcholine, methacholine, carbachol, bethanechol, muscarine, oxotremorine M, oxotremorine, thapsigargin, calcium channel blockers or agonists, nicotine, xanomeline, BuTAC, clozapine, olanzapine, cevimeline, aceclidine, arecoline, tolterodine, rociverine, IQNP, indole alkaloids, himbacine, cyclostellettamines, deriviatives thereof, pro-drugs thereof, and combinations thereof.

A biological sample is preferably cerebrospinal fluid or serum.

Also provided is a composition for distinguishing the magnitude of neurological injury in a subject. An inventive composition illustratively includes a biological sample isolated from a subject suspected of having a damaged nerve cell, the biological sample being a fluid in communication with the nervous system of the subject prior to being isolated from the subject; and at least two added antibodies that specifically and independently bind to at least two biomarkers selected from αII-spectrin, an αII-spectrin breakdown product (SBDP), a ubiquitin carboxyl-terminal hydrolase, and a MAP2 protein.

The subject of the inventive composition is optionally a mammal. Preferably, the subject is a human.

The inventive composition optionally includes a substrate upon which a biomarker is immobilized. The inventive composition optionally includes at least one detectable label that is optionally conjugated to the at least two antibodies. A label is preferably conjugated to a substance that specifically binds to the at least two antibodies.

Also provided is a kit for analyzing cell damage in a subject. An inventive kit illustratively includes a substrate for associating with a biomarker in a biological sample isolated from a subject suspected of having a damaged nerve cell, the biological sample being a fluid in communication with the nervous system of the subject prior to being isolated from the subject; at least two antibodies that specifically and independently bind to at least two biomarkers selected from αII-spectrin, an αII-spectrinbreakdown product (SBDP), a ubiquitin carboxyl-terminal hydrolase, and a MAP2 protein; and instructions for reacting the antibodies with the biological sample or a portion of the biological sample to detect the presence or amount of the markers in the biological sample.

The subject of the inventive kit is optionally a mammal. Preferably, the subject is a human.

The inventive kit optionally includes at least one detectable label that is optionally conjugated to the at least two antibodies. A label is preferably conjugated to a substance that specifically binds to the at least two antibodies.

A process for diagnosing a neurological condition in a subject is provided. The inventive process includes measuring at a first time a quantity of a ubiquitin carboxyl-terminal hydrolase and a quantity of at least one additional neuroactive biomarker in a biological sample obtained from a subject; and comparing the quantity of a ubiquitin carboxyl-terminal hydrolase and the quantity of the at least one additional neuroactive biomarker to baseline levels of a ubiquitin carboxyl-terminal hydrolase and the at least one additional neuroactive biomarker to diagnose a neurological condition of the subject.

The measuring of the inventive process is preferably by immunoassay. Preferably, the sample is cerebrospinal fluid or serum. The one additional neuroactive biomarker is preferably a spectrin breakdown product, MAP-2, SBDP150, SBDP145, or SBDP120, or glial fibrillary acidic protein.

The inventive process optionally comprises measuring a second quantity of ubiquitin carboxyl-terminal hydrolase and a second quantity of the at least one additional neuroactive biomarker at a second time to yield a kinetic profile for ubiquitin carboxyl-terminal hydrolase and the at least one additional neuroactive biomarker. Optionally, the inventive process further includes comparing the quantity of ubiquitin carboxyl-terminal hydrolase and the quantity of the at least one additional neuroactive biomarker between normal levels in the subject to other individuals of the same gender as the subject.

Also provided is a process for diagnosing and treating multiple-organ injury in a subject. The inventive process includes assaying a biological sample from a subject for a plurality of biomarkers; determining a first subtype of first organ injury based on a first ratio of said plurality of biomarkers in said biological sample; determining a second subtype of second organ injury based on second ratio of said plurality of biomarkers in said biological sample; administering at least one therapeutic antagonist effective to inhibit activity of a protein released in response to said first subtype of first organ injury or at least one therapeutic agonist effective to promote activity of a protein released in response to said first subtype of first organ injury; and administering at least one therapeutic antagonist effective to inhibit activity of a protein released in response to said second subtype of second organ injury or at least one therapeutic agonist effective to promote activity of a protein released in response to said second subtype of second organ injury.

The protein of the inventive process is optionally a caspase or a calpain. Preferably, a protein is caspase-3.

Preferably, the plurality of biomarkers are cellular breakdown products associated with injury of at least one of said first and said second organs.

The injury in the inventive process is preferably percussive injury or stroke. The therapeutic is preferably dicyclomine.

Also provided is a process for diagnosis and treatment of a traumatic brain injury in a subject including assaying a subject tissue or fluid for one or more spectrin breakdown products and administering dicyclomine. Preferably the traumatic brain injury is a stroke. More preferably, the injury is ischemic stroke. Optionally, the injury is percussive brain injury.

BRIEF DESCRIPTION OF THE DRAWINGS

**Figure 1** represents quantitative western blotting of UCHL1 in rat CSF following CCI;
**Figure 2** represents quantitative western blotting of UCHL1 in rat CSF following MCAO;
**Figure 3** represents UCHL1 levels in CSF in sham and CCI treated subjects;
**Figure 4** represents UCHL1 levels in CSF following sham, mild MCAO challenge, and severe MCAO challenge;
**Figure 5** represents UCHL1 levels in serum following sham or CCI at various timepoints;
**Figure 6** represents UCHL1 levels in serum following sham, mild MCAO challenge, and severe MCAO challenge;
**Figure 7** represents SBDP145 levels in CSF and serum following sham, mild MCAO challenge, and severe MCAO challenge;
**Figure 8** represents SBDP120 levels in CSF and serum following sham, mild MCAO challenge, and severe MCAO challenge;
**Figure 9** represents MAP2 elevation in CSF and serum following sham, mild MCAO challenge, and severe MCAO challenge;
**Figure 10** represents the effect of dicyclomine on SBDPs in CSF following CCI;
**Figure 11** represents cell death in neuronal cells following CCI in the presence or absence of dicyclomine.

DETAILED DESCRIPTION OF THE PREFERRED EMBODIMENTS

The present invention has utility in the diagnosis and management of abnormal neurological condition. Specifically, the invention has utility in the use of a diagnostic to classify disease or injury subtype, specifically a traumatic brain injury (TBI) subtype, alone or in combination with multi-organ injury and identifying potential therapeutics effective for the particular traumatic brain injury subtype the subject has endured. The invention and as used herein is defined as a theranostic TBI process.

The present theranostic process involves assaying for hyper- or hypo-activation of cellular proteases or combinations thereof to confirm a subtype of brain injury illustratively including MTBI and TBI. An exemplary protein or combination of proteins includes a calpain; ubiquitin carboxyl-terminal esterase such as L1 (UCHL1); additional proteases that result in spectrin breakdown products such as SBDP150, SBDP145, and SBDP120; neuronally-localized intracellular protein MAP-tau; C-tau; MAP2; Poly (ADP-ubose) polymerase (PARP); a collapsin response mediator protein such as protein 2 (CRMP-2); and a caspase such as caspase-3 and the like.

Brain injury occurs through a range of severities in injury inducement, resulting damage, and clinical outcome. Identification of molecular markers capable of distinguishing the severity of injury is essential to determining the type and immediacy of therapeutic or treatment chosen to promote the greatest recovery. Several potential markers have been previously proposed including lactate dehydrogenase, glial fibrillary acid protein, enolase, and S-100B. None of these markers individually posses sufficient specificity or robustness to be clinically successful.

By elucidating biochemical promoter or inhibition signals responsive to brain cells after physical or chemical stresses the efficacy of one or more of a battery of prospectively effective compounds can be elucidated. By way of examples, calcium-activated cytosolic protease calpain is known to be activated in pro-necrotic cell injury while caspases are known to be activated in pro-apoptotic cell injury and are implicated in degrading key structural proteins of brain cells, leading to tissue auto-digestion as part of a TBI cascade.

It is appreciated that in addition to a protease, a breakdown products (BDP) produced by the action of a protease on TBI exposed tissues are also effective as markers in characterizing a subtype of TBI. With this diagnostic information, an agonist or antagonist of a given protease or other compound changing biochemical concentration in response to a TBI is then administered. In the case of a calpain and caspase-3 TBI cascades, administration of calpain and caspase protease inhibitors following TBI offer neuroprotection against both acute and delayed brain cell injury or death associated with the TBI.

Protease activity is optionally regulated by controlling, monitoring, or otherwise altering the expression level or activity of the protein by modulating cellular activities that occur upstream of the desired protein activity. For example, regulation of M1 muscarinic receptor activation is believed to possess numerous downstream cellular effects. These effects are believed to result from the $\alpha_{q/11}$ subunit of the M1 muscarinic receptor G protein dissociating from the $\beta\gamma$ subunit complex following receptor ligand binding. The $\alpha$ subunit activates phospholipase C (PLC) causing the hydrolysis of phosphatidylinositol 4,5-bisphosphate ($PIP_2$) into inositol 1,4,5 trisphosphate ($IP_3$) and diacylglycerol (DG). $IP_3$ stimulates the release of intracellular $Ca^{2+}$ stores. DAG activates PKC that phosphorylates a number of proteins including ion channels and the NMDA receptor complex. The $\beta\gamma$ G-protein subunit inhibits both muscarinic ($I_{K(M)}$) and calcium-regulated ($I_{K(Ca)}$) potassium currents which increases cell excitability by augmenting depolarization (Lyeth, 2001). Several studies provide compelling evidence that $PIP_2$ signal transduction pathways are activated after TBI. Wei and colleagues (Wei et al., 1982) first reported PLC activation immediately following fluid percussion TBI in cats. Later studies in the rat lateral fluid percussion model found elevated levels of $IP_3$ at five minutes post-injury in the ipsilateral hippocampus and for up to 20 minutes after injury in the ipsilateral cortex (Prasad et al., 1994). Subsequent studies measured the phosphodiestric breakdown of membrane $PIP_2$ following fluid percussion TBI in rats and found that concentrations of tissue $PIP_2$ were significantly decreased at times when DG was elevated (Dhillon et al., 1995). Later studies examining the link between muscarinic receptors and TBI-induced activation of the PLC signal transduction pathway found that pharmacological blockade of muscarinic receptors with scopolamine at the time of injury significantly blunts the acute decrease in $PIP_2$ derived fatty acids (Lyeth et al., 1996). Thus, the activation of M1 receptors is associated with enhanced excitatory modulation though elevations in intracellular $Ca^{2+}$, amplification of NMDA receptor sensitivity, and increased neuronal excitability.

For purposes of the subject invention, brain injury is divided into two levels, mild traumatic brain injury (MTBI), and traumatic brain injury (TBI). TBI is defined as an injury that correlates with a two-fold increase or greater of two-fold decrease or greater in molecular marker levels or activities. MTBI is defined and an injury that correlates with less than a two-fold increase or two-fold decrease in molecular marker levels or activities.

As used herein an injury is an alteration in cellular or molecular integrity, activity, level, robustness, state, or other alteration that is traceable to an event. Injury illustratively includes a physical, mechanical, chemical, biological, functional, infectious, or other modulator of cellular or molecular characteristics. An event is illustratively, a physical trauma such as an impact (percussive) or a biological abnormality such as a stroke resulting from either blockade or leakage of a blood vessel. An event is optionally an infection by an infectious agent. A person of skill in the art recognizes numerous equivalent events that are encompassed by the terms injury or event.

An injury is optionally a physical event such as a percussive impact. An impact is the like of a percussive injury such as resulting to a blow to the head that either leaves the cranial structure intact or results in breach thereof. Experimentally, several impact methods are used illustratively including controlled cortical impact (CCI) at a 1.6 mm depression depth, equivalent to severe TBI in human. This method is described in detail by Cox, CD, et al., J Neurotrauma, 2008; 25(11):1355-65. It is appreciated that other experimental methods producing impact trauma are similarly operable.

TBI may also result from stroke. Ischemic stroke is optionally modeled by middle cerebral artery occlusion (MCAO) in rodents. UCHL1 protein levels, for example, are increased following mild MCAO which is further increased following severe MCAO challenge. Mild MCAO challenge may result in an increase of protein levels within two hours that is transient and returns to control levels within 24 hours. In contrast, severe MCAO challenge results in an increase in protein levels within two hours following injury and may be much more persistent demonstrating statistically significant levels out to 72 hours or more.

Molecular markers are optionally termed biomarkers and the phrases are used interchangeably herein. A biomarker is a cell, protein, nucleic acid, steroid, fatty acid, metabolite, or other differentiator useful for measurement of biological activity or response. Biomarkers operable herein to distinguish the MTBI and TBI illustratively include: ubiquitin carboxyl-terminal esterase, ubiquitin carboxy-terminal hydrolase, spectrin breakdown product(s), a neuronally-localized intracellular protein, MAP-tau, C-tau, MAP2, poly (ADP-ribose) polymerase (PARP), collapsin response mediator protein, UCHL1, Annexin A11, Aldehyde dehydrogenase family 7, Cofilin 1, Profilin 1, http://www.mcponline.org/cgi/external ref?access num=XP 227366&link type=GENα-Enolase (non-neural enolase), Enolase1 protein, Glyceraldehyde-3-phosphate dehydrogenase, Hexokinase 1, Aconitase 2, mitochondrial, Acetyl-CoA synthetase 2, Neuronal protein 22, Phosphoglycerate kinase 2, Phosphoglycerate kinase 1, Hsc70-ps1, Glutamate dehydrogenase 1, Aldolase A, Aldolase C, fructose-biphosphate, Dimethylarginine dimethylaminohydrolase 1, Microtubule-associated protein 2, Carbonic anhydrase, ADP-ribosylation factor 3, Transferrin, Liver regeneration-related protein, Hemoglobin α-chain, Hemoglobin β chain, Liver regeneration-related protein, Fetuin β, 3-Oxoacid-CoA transferase, Malate dehydrogenase 1, NAD (soluble), Lactate dehydrogenase B, Malate dehydrogenase, mitochondrial, Carboxylesterase E1 precursor, Serine protease inhibitor α1, Haptoglobin, Ubiquitin carboxyl-terminal hydrolase L1, Serine protease inhibitor 2a, T-kininogen, α1 major acute phase protein, http://www.mcponline.org/cgi/external ref?access num=AAH85359&link type=GENAlbumin, http://www.mcponline.or2/cgi/external ref?access num=NP 001009628&link type=GENα1 major acute phase protein prepeptide, Murinoglobulin 1 homolog, Group-specific component protein, Guanosine diphosphate dissociation inhibitor 1, Collapsin response mediator protein 2, Murinoglobulin 1 homolog, Ferroxidase, Ceruloplasmin, Spectrin α-chain, brain, C-reactive protein, Brain creatine kinase, Proteasome subunit, $\alpha$type 7, 14-3-3 protein, Synaptotagmin, subtypes thereof, fragments thereof, breakdown product thereof, or combinations thereof. Other potential biomarkers illustratively include those identified by Kobeissy, FH, et al, Molecular & Cellular Proteomics, 2006; 5:1887-1898, the contents of which are incorporated herein by reference, or others known in the art.

Preferably, a biomarker is selective for detecting or diagnosing a neurological conditions such as brain injury and the like. More preferably, a biomarker is both specific and effective for the detection and distinguishing levels of TBI. Such biomarkers are optionally termed neuroactive biomarkers. In preferred embodiments biomarkers operable herein illustratively are spectrin breakdown products (SBDP) SBDP150, SBDP150i, SBDP145 (calpain mediated acute neural necrosis), SBDP120 (caspase mediated delayed neural apoptosis), UCHL1 (neuronal cell body damage marker), glial fibrillary acidic protein (GFAP), and MAP-2 dendritic cell injury associated marker.

It is appreciated that the temporal nature of biomarker presence or activity is operable as an indicator or distinguisher of TBI subtype. In a non-limiting example, the severity of experimental middle cerebral artery occlusion (MCAO) correlates with the temporal maintenance of UCHL1 in CSF. MCAO of 30 minutes produces transient UCHL1 levels peaking at 6 hours and rapidly decreasing, whereas MCAO of 2 hours produces sustained UCHL1 levels for as many as three days. Similarly, the prevalence of other biomarkers at various timepoints following injury is operable to distinguish TBI subtype.

Biomarker analyses are preferably performed using biological samples or fluids. Illustrative biological samples operable herein illustratively include, cells, tissues, cerebral spinal fluid (CSF), artificial CSF, whole blood, serum, plasma, cytosolic fluid, urine, feces, stomach fluids, digestive fluids, saliva, nasal or other airway fluid, vaginal fluids, semen, buffered saline, saline, water, or other biological fluid recognized in the art.

In addition to increased cell expression, biomarkers also appear in biological fluids in communication with injured cells. Obtaining biological fluids such as cerebrospinal fluid (CSF), blood, plasma, serum, saliva and urine, from a subject is typically much less invasive and traumatizing than obtaining a solid tissue biopsy sample. Thus, samples that are biological fluids are preferred for use in the invention. CSF, in particular, is preferred for detecting nerve damage in a subject as it is in immediate contact with the nervous system and is readily obtainable. It is appreciated serum is a preferred biological sample as it is much more easily obtainable and presents much less risk of further injury or side-effect to a donating subject.

Biological Samples are preferably obtained from one or more subjects. A biological sample is obtained from a subject by conventional techniques. For example, CSF is obtained by lumbar puncture. Blood is obtained by venipuncture, while plasma and serum are obtained by fractionating whole blood according to known methods. Surgical techniques for obtaining solid tissue samples are well known in the art. For example, methods for obtaining a nervous system tissue sample are described in standard neurosurgery texts such as Atlas of Neurosurgery: Basic Approaches to Cranial and Vascular Procedures, by F. Meyer, Churchill Livingstone, 1999; Stereotactic and Image Directed Surgery of Brain Tumors, 1st ed., by David G. T. Thomas, WB Saunders Co., 1993; and Cranial Microsurgery: Approaches and Techniques, by L. N. Sekhar and E. De Oliveira, 1st ed., Thieme Medical Publishing, 1999. Methods for obtaining and analyzing brain tissue are also described in Belay et al., Arch. Neurol. 58: 1673-1678 (2001); and Seijo et al., J. Clin. Microbiol. 38: 3892-3895 (2000).

After insult, nerve cells in *in vitro* culture or *in situ* in a subject express altered levels or activities of one or more proteins than do such cells not subjected to the insult. Thus, samples that contain nerve cells, e.g., a biopsy of a central nervous system or peripheral nervous system tissue are suitable biological samples for use in the invention. In addition to nerve cells, however, other cells express illustratively $\alpha$II-spectrin including, for example, cardiomyocytes, myocytes in skeletal muscles, hepatocytes, kidney cells and cells in testis. A biological sample including such cells or fluid secreted from these cells might also be used in an adaptation of the inventive methods to determine and/or characterize an injury to such non-nerve cells.

A subject as used herein illustratively includes a dog, a cat, a horse, a cow, a pig, a sheep, a goat, a chicken, non-human primate, a human, a rat, guinea pig, hamster, and a mouse. Because the present invention preferably relates to human subjects, a preferred subject for the methods of the invention is a human being.

Subjects who most benefit from the present invention are those suspected of having or at risk for developing abnormal neurological conditions or injury, such as victims of brain injury caused by traumatic insults (e.g., gunshot wounds, automobile accidents, sports accidents, shaken baby syndrome, other percussive injuries), ischemic events (e.g., stroke, cerebral hemorrhage, cardiac arrest), neurodegenerative disorders (such as Alzheimer's, Huntington's, and Parkinson's diseases; prion-related disease; other forms of dementia), epilepsy, substance abuse (e.g., from amphetamines, Ecstasy/MDMA, or ethanol), and peripheral nervous system pathologies such as diabetic neuropathy, chemotherapy-induced neuropathy and neuropathic pain.

To provide correlations between a neurological condition and measured quantities of neuroactive biomarkers CSF or serum are preferable biological fluids. Illustratively, samples of CSF or serum are collected from subjects with the samples being subjected to measurement of neuroactive biomarkers. The subjects vary in neurological condition. Detected levels of one or more biomarkers are then correlated with either recognized or standardized baseline levels or optionally CT scan results as well as GCS scoring. Based on these results, an inventive assay is optionally developed and validated (Lee et al., Pharmacological Research 23:312-328, 2006). It is appreciated that neuroactive biomarkers, in addition to being obtained from CSF and serum, are also readily obtained from blood, plasma, saliva, urine, as well as solid tissue biopsy. While CSF is a preferred sampling fluid owing to direct contact with the nervous system, it is appreciated that other biological fluids have advantages in being sampled for other purposes and therefore allow for inventive determination of neurological condition as part of a battery of tests performed on a single sample such as blood, plasma, serum, saliva or urine.

Baseline levels of several biomarkers are those levels obtained in the target biological sample in the species of desired subject in the absence of a known neurological condition. These levels need not be expressed in hard concentrations, but may instead be known from parallel control experiments and expressed in terms of fluorescent units, density units, and the like. Typically, in the absence of a neurological condition SBDPs are present in biological samples at a negligible amount. However, UCHL1 is a highly abundant protein in neurons. Determining the baseline levels of UCHL1 in neurons of particular species is well within the skill of the art. Similarly, determining the concentration of baseline levels of MAP2 is well within the skill of the art.

As used herein the term "diagnosing" means recognizing the presence or absence of a neurological or other condition such as an injury or disease. Diagnosing is optionally referred to as the result of an assay wherein a particular ratio or level of a biomarker is detected or is absent.

As used herein a "ratio" is either a positive ratio wherein the level of the target is greater than the target in a second sample or relative to a known or recognized baseline level of the same target. A negative ratio describes the level of the target as lower than the target in a second sample or relative to a known or recognized baseline level of the same target. A neutral ratio describes no observed change in target biomarker.

As used herein the term "administering" is delivery of a therapeutic to a subject. The therapeutic is administered by a route determined to be appropriate for a particular subject by one skilled in the art. For example, the therapeutic is administered orally, parenterally (for example, intravenously), by intramuscular injection, by intraperitoneal injection, intratumorally, by inhalation, or transdermally. The exact amount of therapeutic required will vary from subject to subject, depending on the age, weight and general condition of the subject, the severity of the neurological condition that is being treated, the particular therapeutic used, its mode of administration, and the like. An appropriate amount may be determined by one of ordinary skill in the art using only routine experimentation given the teachings herein or by knowledge in the art without undue experimentation.

An exemplary process for detecting the presence or absence of one or more neuroactive biomarkers in a biological sample involves obtaining a biological sample from a subject, such as a human, contacting the biological sample with a compound or an agent capable of detecting of the biomarker being analyzed, illustratively including an antibody or aptamer, and analyzing binding of the compound or agent to the sample after washing. It is appreciated that other detection methods are similarly operable illustratively contact with a protein or nucleic acid specific stain. In the case of antibody or aptamer, those samples having specifically bound compound or agent express of the marker being analyzed.

The present invention is optionally described with respect to UCHL1 or SBDP. It is appreciated that these biomarkers are presented for illustrative purposes only and are not meant to imply expressly or otherwise that the scope of the present invention is limited to UCHL1 or SBDPs.

An inventive process can be used to detect UCHL1 and one or more other neuroactive biomarkers in a biological sample *in vitro,* as well as *in vivo.* The quantity of expression of UCHL1 or one or more other neuroactive biomarkers in a sample is compared with appropriate controls such as a first sample known to express detectable levels of the marker being analyzed (positive control) and a second sample known to not express detectable levels of the marker being analyzed (a negative control). For example, *in vitro* techniques for detection of a marker include enzyme linked immunosorbent assays (ELISAs), radioimmuno assay, radioassay, western blot, Southern blot, northern blot, immunoprecipitation, immunofluorescence, mass spectrometry, RT-PCR, PCR, liquid chromatography, high performance liquid chromatography, enzyme activity assay, cellular assay, positron emission tomography, mass spectroscopy, combinations thereof, or other technique known in the art. Furthermore, *in vivo* techniques for detection of a marker include introducing a labeled agent that specifically binds the marker into a biological sample or test subject. For example, the agent can be labeled with a radioactive marker whose presence and location in a biological sample or test subject can be detected by standard imaging techniques.

Any suitable molecule that can specifically bind UCHL1 or other biomarker and any suitable molecule that specifically binds one or more other neuroactive biomarkers are operative in the invention. A preferred agent for detecting UCHL1 or the one or more other neuroactive biomarkers is an antibody capable of binding to the biomarker being analyzed, preferably an antibody is conjugated with a detectable label. Such antibodies can be polyclonal or monoclonal. An intact antibody, a fragment thereof (e.g., Fab or F(ab')$_2$), or an engineered variant thereof (e.g., sFv) can also be used. Such antibodies can be of any immunoglobulin class including IgG, IgM, IgE, IgA, IgD and any subclass thereof. Antibodies operable herein are optionally monoclonal or polyclonal.

An antibody is optionally labeled. A person of ordinary skill in the art recognizes numerous labels operable herein. Labels and labeling kits are commercially available optionally from Invitrogen Corp, Carlsbad, CA. Labels illustratively include, fluorescent labels, biotin, peroxidase, radionucleotides, or other label known in the art.

Antibody-based assays are preferred for analyzing a biological sample for the presence of UCHL1 and one or more other neuroactive biomarkers. Suitable western blotting methods are described below in the examples section. For more rapid analysis (as may be important in emergency medical situations), immunosorbent assays (e.g., ELISA and RIA) and immunoprecipitation assays may be used. As one example, the biological sample or a portion thereof is immobilized on a substrate, such as a membrane made of nitrocellulose or PVDF; or a rigid substrate made of polystyrene or other plastic polymer such as a microtiter plate, and the substrate is contacted with an antibody that specifically bind UCHL1, or one of the other neuroactive biomarkers under conditions that allow binding of antibody to the biomarker being analyzed. After washing, the presence of the antibody on the substrate indicates that the sample contained the marker being assessed. If the antibody is directly conjugated with a detectable label, such as an enzyme, fluorophore, or radioisotope, the label presence is optionally detected by examining the substrate for the detectable label. Alternatively, a detectably labeled secondary antibody that binds the marker-specific antibody is added to the substrate. The presence of detectable label on the substrate after washing indicates that the sample contained the marker.

Numerous permutations of these basic immunoassays are also operative in the invention. These include the biomarker-specific antibody, as opposed to the sample being immobilized on a substrate, and the substrate is contacted with UCHL1 or another neuroactive biomarker conjugated with a detectable label under conditions that cause binding of antibody to the labeled marker. The substrate is then contacted with a sample under conditions that allow binding of the marker being analyzed to the antibody. A reduction in the amount of detectable label on the substrate after washing indicates that the sample contained the marker.

Although antibodies are preferred for use in the invention because of their extensive characterization, any other suitable agent (e.g., a peptide, an aptamer, or a small organic molecule) that specifically binds UCHL1 or another neuroactive biomarker is optionally used in place of the antibody in the above-described immunoassays. For example, an aptamer that specifically binds all spectrin and/or one or more of its SBDPs might be used. Aptamers are nucleic acid-based molecules that bind specific ligands. Methods for making aptamers with a particular binding specificity are known as detailed in U.S. Patent Nos. 5,475,096; 5,670,637; 5,696,249; 5,270,163; 5,707,796; 5,595,877; 5,660,985; 5,567,588; 5,683,867; 5,637,459; and 6,011,020.

A myriad of detectable labels that are operative in a diagnostic assay for biomarker expression are known in the art. Agents used in methods for detecting UCHL1 or another neuroactive biomarker are conjugated to a detectable label, e.g., an enzyme such as horseradish peroxidase. Agents labeled with horseradish peroxidase can be detected by adding an appropriate substrate that produces a color change in the presence of horseradish peroxidase. Several other detectable labels that may be used are known. Common examples of these include alkaline phosphatase, horseradish peroxidase, fluorescent compounds, luminescent compounds, colloidal gold, magnetic particles, biotin, radioisotopes, and other enzymes. It is appreciated that a primary/secondary antibody system is optionally used to detect one or more biomarkers. A primary antibody that specifically recognizes one or more biomarkers is exposed to a biological sample that may contain the biomarker of interest. A secondary antibody with an appropriate label that recognizes the species or isotype of the primary antibody is then contacted with the sample such that specific detection of the one or more biomarkers in the sample is achieved.

The present invention employs a step of correlating the presence or amount of UCHL1 and one or more other neuroactive biomarker in a biological sample with the severity and/or type of nerve cell injury. The amount of UCHL1 and one or more other neuroactive biomarkers in the biological sample is associated with neurological condition for traumatic brain injury as detailed in the examples. The results of an inventive assay to synergistically measure UCHL1 and one or more other neuroactive biomarkers can help a physician determine the type and severity of injury with implications as to the types of cells that have been compromised. These results are in agreement with CT scan and GCS results, yet are quantitative, obtained more rapidly, and at far lower cost.

The present invention provides a step of comparing the quantity of UCHL1 and optionally the amount of at least one other neuroactive biomarker to normal levels or one or each to determine the neurological condition of the subject. It is appreciated that selection of additional biomarkers allows one to identify the types of nerve cells implicated in an abnormal neurological condition as well as the nature of cell death in the case of an axonal injury marker, namely an SBDP. The practice of an inventive process provides a test which can help a physician determine suitable therapeutic(s) to administer for optimal benefit of the subject. While the subsequently provided data found in the examples is provided with respect to a full spectrum of traumatic brain injury, it is appreciated that these results are applicable to ischemic events, neurodegenerative disorders, prison related disease, epilepsy, chemical etiology and peripheral nervous system pathologies. A gender difference may be present in abnormal subject neurological condition.

An assay for analyzing cell damage in a subject is also provided. The assay includes: (a) a substrate for holding a sample isolated from a subject suspected of having a damaged nerve cell, the sample being a fluid in communication with the nervous system of the subject prior to being isolated from the subject; (b) a UCHL1 specific binding agent; (c) optionally a binding agent specific for another neuroactive biomarker; and (d) printed instructions for reacting: the UCHL1 agent with the biological sample or a portion of the biological sample to detect the presence or amount of UCHL1, and the agent specific for another neuroactive biomarker with the biological sample or a portion of the biological sample to detect the presence or amount of the at least one biomarker in the biological sample. The inventive assay can be used to detect neurological condition for financial remuneration.

The assay optionally includes a detectable label such as one conjugated to the agent, or one conjugated to a substance that specifically binds to the agent, such as a secondary antibody.

A theranostic process of the present invention optionally includes the presence of one or more therapeutic agents that may alter one or more characteristics of a target biomarker. A therapeutic is optionally serves as an agonist or antagonist of a target biomarker or upstream effector of a biomarker. A therapeutic optionally affects a downstream function of a biomarker. For example, Acetylcholine (Ach) plays a role in pathological neuronal excitation and TBI-induced muscarinic cholinergic receptor activation may contribute to excitotoxic processes. As such, biomarkers optionally include levels or activity of Ach or muscarinic receptors. Optionally, an operable biomarker is a molecule, protein, nucleic acid or other that is effected by the activity of muscarinic receptor(s). As such, therapeutics operable in the subject invention illustratively include those that modulate various aspects of muscarinic cholinergic receptor activation.

Specific muscarinic receptors operable as therapeutic targets or modulators of therapeutic targets include the $M_1$, $M_2$, $M_3$, $M_4$, and $M_5$ muscarinic receptors.

The suitability of the muscarinic cholinergic receptor pathway in detecting and treating TBI arises from studies that demonstrated elevated ACh in brain cerebrospinal fluid (CSF) following experimental TBI (Gorman et al., 1989; Lyeth et al., 1993a) and ischemia (Kumagae and Matsui, 1991), as well as the injurious nature of high levels of muscarinic cholinergic receptor activation through application of cholinomimetics (Olney et al., 1983; Turski et al., 1983). Furthermore, acute administration of muscarinic antagonists improves behavioral recovery following experimental TBI (Lyeth et al., 1988a; Lyeth et al., 1988b; Lyeth and Hayes, 1992; Lyeth et al., 1993b; Robinson et al., 1990).

A therapeutic operable in the subject invention is illustratively any molecule, compound, family, extract, solution, drug, pro-drug, or other mechanism that is operable for changing, preferably improving, therapeutic outcome of a subject at risk for or victim of a neuronal injury such as TBI or MTBI. A therapeutic is optionally a muscarinic cholinergic receptor modulator such as an agonist or antagonist. An agonist or antagonist may by direct or indirect. An indirect agonist or antagonist is optionally a molecule that breaks down or synthesizes acetylcholine or other muscarinic receptor related molecule illustratively, molecules currently used for the treatment of Alzheimer's disease. Cholinic mimetics or similar molecules are operable herein. An exemplary list of therapeutics operable herein include: dicyclomine, scoplamine, milameline, N-methyl-4-piperidinylbenzilate NMP, pilocarpine, pirenzepine, acetylcholine, methacholine, carbachol, bethanechol, muscarine, oxotremorine M, oxotremorine, thapsigargin, calcium channel blockers or agonists, nicotine, xanomeline, BuTAC, clozapine, olanzapine, cevimeline, aceclidine, arecoline, tolterodine, rocivenne, IQNP, indole alkaloids, himbacine, cyclostellettamines, deriviatives thereof, pro-drugs thereof, and combinations thereof. A therapeutic is optionally a molecule operable to alter the level of or activity of a calpain or caspase. Such molecules and their administration are known in the art.

An inventive theranostic process illustratively includes a process for diagnosing a neurological condition in a subject, treating a subject with a neurological condition, or both. In a preferred embodiment an inventive process illustratively includes obtaining a biological sample from a subject. The biological sample is assayed by mechanisms known in the art for detecting or identifying the presence of one or more biomarkers present in the biological sample. Based on the amount or presence of a target biomarker in a biological sample, a ratio of one or more biomarkers is optionally calculated. The ratio is optionally the level of one or more biomarkers relative to the level of another biomarker in the same or a parallel sample, or the ratio of the quantity of the biomarker to a measured or previously established baseline level of the same biomarker in a subject known to be free of a pathological neurological condition. The ratio allows for the diagnosis of a neurological condition in the subject. An inventive process also administers a therapeutic to the subject that will either directly or indirectly alter the ratio of one or more biomarkers.

An inventive process is also provided for diagnosing and treating a multiple-organ injury. Multiple organs illustratively include subsets of neurological tissue such as brain, spinal cord and the like, or specific regions of the brain such as cortex, hippocampus and the like. Multiple injuries illustratively include apoptotic cell death which is detectable by the presence of caspase induced SBDPs, and oncotic cell death which is detectable by the presence of calpain induced SBDPs. The inventive process illustratively includes assaying for a plurality of biomarkers in a biological sample obtained from a subject wherein the biological was optionally in fluidic contact with an organ suspected of having undergone injury or control organ when the biological sample was obtained from the subject. The inventive process determines a first subtype of organ injury in based on a first ratio of a plurality of biomarkers. The inventive process also determines a second subtype of a second organ injury based on a second ration of the plurality of biomarkers in the biological sample. The ratios are illustratively determined by processes described herein or known in the art.

Treatment of a multiple organ injury in the inventive process is illustratively achieved by administering to a subject at least one therapeutic antagonist or agonist effective to modulate the activity of a protein whose activity is altered in response to the first organ injury, and administering at least one therapeutic agonist or antagonist effective to modulate the activity of a protein whose activity is altered in response to a second organ injury.

The subject invention illustratively includes a composition for distinguishing the magnitude of a neurological condition in a subject. An inventive composition is either a agent entity or a mixture of multiple agents. In a preferred embodiment a composition is a mixture. The mixture optionally contains a biological sample derived from a subject. The subject is optionally suspected of having a neurological condition. The biological sample in communication with the nervous system of the subject prior to being isolated from the subject. In inventive composition also contains at least two primary agents, preferably antibodies, that specifically and independently bind to at least two biomarkers that may be present in the biological sample. In a preferred embodiment the first primary agent is in antibody that specifically binds a ubiquitin carboxyl-terminal hydrolase, preferably UCHL1. A second primary agent is preferably an antibody that specifically binds a spectrin breakdown product.

The agents of the inventive composition are optionally mobilized or otherwise in contact with a substrate. The inventive teachings are also preferably labeled with at least one detectable label. In a preferred embodiment the detectable label on each agent is unique and independently detectable. Optionally a secondary agent specific for detecting or binding to the primary agent is labeled with at least one detectable label. In the nonlimiting example the primary agent is a rabbit derived antibody. A secondary agent is optionally an antibody specific for a rabbit derived primary antibody. Mechanisms of detecting antibody binding to an antigen are well known in the art, and a person of ordinary skill in the art readily envisions numerous methods and agents suitable for detecting antigens or biomarkers in a biological sample.

The kit is also provided that encompasses a substrate suitable for associating with the target biomarker in a biological sample. The biological sample is optionally provided with the kit or is obtained by a practitioner for use with an inventive kit. An inventive kit also includes at least two antibodies that specifically and independently bind to at least two biomarkers. The antibodies preferably distinguish between the two biomarkers. Preferably, a first antibody is specific and independent for binding and detecting a first biomarker. A second antibody is specific and independent for binding and detecting a second biomarker. In this way the presence or absence of multiple biomarkers in a single biological sample can be determined or distinguished. In a preferred embodiment target Biomarkers in the biological sample illustratively include all-spectrin, an $\alpha$II-spectrin breakdown product (SBDP), a ubiquitin carboxyl-terminal hydrolase, and a MAP2 protein. An inventive kit also includes instructions for reacting the antibodies with the biological sample or a portion of the biological sample so as to detect the presence of or amount of the biomarkers in the biological sample.

In the kit, the biological sample can be CSF or blood, and the agent can be an antibody, aptamer, or other molecule that specifically binds at least one of all spectrin, all-spectrin breakdown product (SBDP), a ubiquitin carboxyl-terminal hydrolase, and a MAP2 protein. Suitable agents are described above. The kit can also include a detectable label such as one conjugated to the agent, or one conjugated to a substance that specifically binds to the agent (e.g., a secondary antibody).

The invention employs a step of correlating the presence or amount of a biomarker in a biological sample with the severity and/or type of nerve cell (or other biomarker-expressing cell) injury. The amount of biomarker(s) in the biological sample directly relates to severity of nerve tissue injury as a more severe injury damages a greater number of nerve cells which in turn causes a larger amount of biomarker(s) to accumulate in the biological sample (e.g., CSF; serum). Whether a nerve cell injury triggers an apoptotic and/or necrotic type of cell death can also be determined by examining the SBDPs present in the biological sample. Necrotic cell death preferentially activates calpain, whereas apoptotic cell death preferentially activates caspase-3. Because calpain and caspase-3 SBDPs can be distinguished, measurement of these markers indicates the type of cell damage in the subject. For example, necrosis-induced calpain activation results in the production of SBDP150 and SBDP145; apoptosis-induced caspase-3 activation results in the production of SBDP150i and SBDP120; and activation of both pathways results in the production of all four markers. Also, the level of or kinetic extent of UCHL1 present in a biological sample may optionally distinguish mild injury from a more severe injury. In an illustrative example, severe MCAO (2h) produces increased UCHL1 in both CSF and Serum relative to mild challenge (30 min) while both produce UCHL1 levels in excess of uninjured subjects. Moreover, the persistence or kinetic extent of the markers in a biological sample is indicative of the severity of the injury with greater injury indicating increases persistence of UCHL1 or SBDP in the subject that is measured by an inventive process in biological samples taken at several timepoints following injury.

The results of such a test can help a physician determine whether the administration a particular therapeutic such as calpain and/or caspase inhibitors or muscarinic cholinergic receptor antagonists might be of benefit to a patient. This application may be especially important in detecting age and gender difference in cell death mechanism.

Methods involving conventional biological techniques are described herein. Such techniques are generally known in the art and are described in detail in methodology treatises such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992.

Various aspects of the present invention are illustrated by the following non-limiting examples. The examples are for illustrative purposes and are not a limitation on any practice of the present invention. It will be understood that variations and modifications can be made without departing from the spirit and scope of the invention. While the examples are generally directed to mammalian tissue, specifically, analyses of rat tissue, a person having ordinary skill in the art recognizes that similar techniques and other techniques know in the art readily translate the examples to other mammals such as humans. Reagents illustrated herein are commonly cross reactive between mammalian species or alternative reagents with similar properties are commercially available, and a person of ordinary skill in the art readily understands where such reagents may be obtained.

Example 1:

Materials for Biomarker Analyses. Sodium bicarbonate, (Sigma Cat #: C-3041), blocking buffer (Startingblock T20-TBS) (Pierce Cat#: 37543), Tris buffered saline with Tween 20 (TBST; Sigma Cat #: T-9039). Phosphate buffered saline (PBS; Sigma Cat #: P-3813); Tween 20 (Sigma Cat #: P5927); Ultra TMB ELISA (Pierce Cat #: 34028); and Nunc maxisorp ELISA plates (Fisher). Monoclonal and polyclonal UCH-L1 antibodies are made in-house or are obtained from Santa Cruz Biotechnology, Santa Cruz, CA. Antibodies directed to $\alpha$-II spectrin and breakdown products as well as to MAP2 are available from Santa Cruz Biotechnology, Santa Cruz, CA. Labels for antibodies of numerous subtypes are available from Invitrogen, Corp., Carlsbad, CA. Protein concentrations in biological samples are determined using bicinchoninic acid microprotein assays (Pierce Inc., Rockford, IL, USA) with albumin standards. All other necessary reagents and materials are known to those of skill in the art and are readily ascertainable.

Example 2:

*In vivo* model of TBI injury model: A controlled cortical impact (CCI) device is used to model TBI on rats as previously described (Pike et al, 1998). Adult male (280-300 g) Sprague-Dawley rats (Harlan: Indianapolis, IN) are anesthetized with 4% isoflurane in a carrier gas of 1:1 $O_2/N_2O$ (4 min.) and maintained in 2.5% isoflurane in the same carrier gas. Core body temperature is monitored continuously by a rectal thermistor probe and maintained at $37\pm1°C$ by placing an adjustable temperature controlled heating pad beneath the rats. Animals are mounted in a stereotactic frame in a prone position and secured by ear and incisor bars. Following a midline cranial incision and reflection of the soft tissues, a unilateral (ipsilateral to site of impact) craniotomy (7 mm diameter) is performed adjacent to the central suture, midway between bregma and lambda. The dura mater is kept intact over the cortex. Brain trauma is produced by impacting the right (ipsilateral) cortex with a 5 mm diameter aluminum impactor tip (housed in a pneumatic cylinder) at a velocity of 3.5 m/s with a 1.6 mm compression and 150 ms dwell time. Sham-injured control animals are subjected to identical surgical procedures but do not receive the impact injury. Appropriate pre- and post-injury management is preformed to insure compliance with guidelines set forth by the University of Florida Institutional Animal Care and Use Committee and the National Institutes of Health guidelines detailed in the Guide for the Care and Use of Laboratory Animals. In addition, research is conducted in compliance with the Animal Welfare Act and other federal statutes and regulations relating to animals and experiments involving animals and adhered to principles stated in the "Guide for the Care and Use of Laboratory Animals, NRC Publication, 1996 edition."

Example 3:

Middle cerebral artery occlusion (MCAO) injury model: Rats are incubated under isoflurane anesthesia (5% isoflurane via induction chamber followed by 2% isoflurane via nose cone), the right common carotid artery (CCA) of the rat is exposed at the external and internal carotid artery (ECA and ICA) bifurcation level with a midline neck incision. The ICA is followed rostrally to the pterygopalatine branch and the ECA is ligated and cut at its lingual and maxillary branches. A 3-0 nylon suture is then introduced into the ICA via an incision on the ECA stump (the suture's path was visually monitored through the vessel wall) and advanced through the carotid canal approximately 20 mm from the carotid bifurcation until it becomes lodged in the narrowing of the anterior cerebral artery blocking the origin of the middle cerebral artery. The skin incision is then closed and the endovascular suture left in place for 30 minutes or 2 hours. Afterwards the rat is briefly reanesthetized and the suture filament is retracted to allow reperfusion. For sham MCAO surgeries, the same procedure is followed, but the filament is advanced only 10 mm beyond the internal-external carotid bifurcation and is left in place until the rat is sacrificed. During all surgical procedures, animals are maintained at $37 \pm 1°C$ by a homeothermic heating blanket (Harvard Apparatus, Holliston, MA, U.S.A.). It is important to note that at the conclusion of each experiment, if the rat brains show pathologic evidence of subarachnoid hemorrhage upon necropsy they are excluded from the study. Appropriate pre- and post-injury management is preformed to insure compliance with all animal care and use guidelines.

**Example 4:**

Tissue and Sample Preparation: At the appropriate time points (2, 6, 24 hours and 2, 3, 5 days) after injury, animals are anesthetized and immediately sacrificed by decapitation. Brains are quickly removed, rinsed with ice cold PBS and halved. The right hemisphere (cerebrocortex around the impact area and hippocampus) is rapidly dissected, rinsed in ice cold PBS, snap-frozen in liquid nitrogen, and stored at -80°C until used. For immunohistochemistry, brains are quick frozen in dry ice slurry, sectioned via cryostat (20 $\mu$m) onto SUPERFROST PLUS GOLD® (Fisher Scientific) slides, and then stored at -80°C until used. For the left hemisphere, the same tissue as the right side is collected. For Western blot analysis, the brain samples are pulverized with a small mortar and pestle set over dry ice to a fine powder. The pulverized brain tissue powder is then lysed for 90 min at 4°C in a buffer of 50 mM Tris (pH 7.4), 5 mM EDTA, 1% (v/v) Triton X-100, 1 mM DTT, 1x protease inhibitor cocktail (Roche Biochemicals). The brain lysates are then centrifuged at 15,000xg for 5 min at 4°C to clear and remove insoluble debris, snap-frozen, and stored at -80°C until used.
For gel electrophoresis and electroblotting, cleared CSF samples (7 $\mu$l) are prepared for sodium dodecyl sulfate-polyacrylamide gel electrophoresis (SDS-PAGE) with a 2X loading buffer containing 0.25 M Tris (pH 6.8), 0.2 M DTT, 8% SDS, 0.02% bromophenol blue, and 20% glycerol in distilled $H_2O$. Twenty micrograms (20 $\mu$g) of protein per lane are routinely resolved by SDS-PAGE on 10-20% Tris/glycine gels (Invitrogen, Cat #EC61352) at 130 V for 2 hours. Following electrophoresis, separated proteins are laterally transferred to polyvinylidene fluoride (PVDF) membranes in a transfer buffer containing 39 mM glycine, 48 mM Tris-HCl (pH 8.3), and 5% methanol at a constant voltage of 20 V for 2 hours at ambient temperature in a semi-dry transfer unit (Bio-Rad). After electro-transfer, the membranes are blocked for 1 hour at ambient temperature in 5% non-fat milk in TBS and 0.05% Tween-2 (TBST) then are incubated with the primary polyclonal UCHL1 antibody in TBST with 5% non-fat milk at 1:2000 dilution as recommended by the manufacturer at 4°C overnight. This is followed by three washes with TBST, a 2 hour incubation at ambient temperature with a biotinylated linked secondary antibody (Amersham, Cat # RPN1177v1), and a 30 min incubation with Streptavidin-conjugated alkaline phosphatase (BCIP/NBT reagent: KPL, Cat # 50-81-08). Molecular weights of intact UCHL1 proteins are assessed using rainbow colored molecular weight standards (Amersham, Cat # RPN800V). Semi-quantitative evaluation of intact UCHL1 proteins levels is performed via computer-assisted densitometric scanning (Epson XL3500 scanner) and image analysis with ImageJ software (NIH).

**Example 5:**

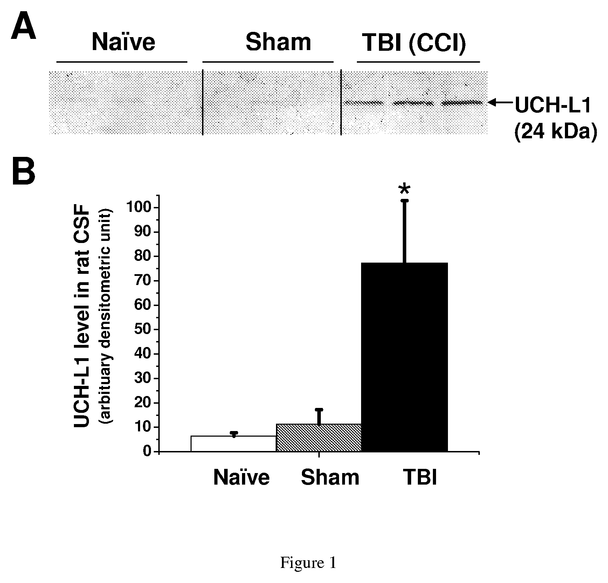

UCHL1 expression is specific to neural tissue. Tissue specificity of UCHL1 is analyzed by as in Example 4 to identify tissue specific expression. UCHL1 is expressed primarily in brain (FIG. 1A) and at low levels in testis. UCHL1 is present across all brain regions examined with reduced levels in the cerebellum and pons (FIG. 1B).

**Example 6:**

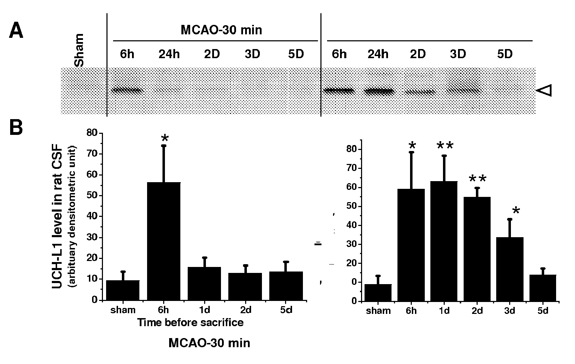

UCHL1 is increased in CSF following MCAO challenge. Subjects are subjected to MCAO challenge as described in Example 3 and CSF samples analyzed by quantitative western blotting. UCHL1 protein is readily detectable after injury at statically significant levels above the amounts of UCHL1 in sham treated samples (FIGs. 2A, B). These UCHL1 levels are transiently elevated (at 6 h) after mild ischemia (30 min MCAO) followed by reperfusion, while levels are sustained from 6 to 72 h after a more severe (2 h MCAO) ischemia (FIGs. 2A, B).

**Example 7:**

ELISA readily identifies UCHL1 levels in both CSF and Serum. ELISA is used to more rapidly and readily detect and quantitate UCHL1 in biological samples. For a UCHL1 sandwich ELISA (swELISA), 96-well plates are coated with 100 $\mu$l/well capture antibody (500 ng/well purified rabbit anti-UCHL1, made in-house) in 0.1 M sodium bicarbonate, pH 9.2. Plates are incubated overnight at 4°C, emptied and 300 $\mu$l/well blocking buffer (Startingblock T20-TBS) is added and incubated for 30 min at ambient temperature with gentle shaking. This is followed by either the addition of the antigen standard (recombinant UCHL1) for standard curve (0.05 - 50 ng/well) or samples (3-10 $\mu$l CSF) in sample diluent (total volume 100 $\mu$l/well). The plate is incubated for 2 hours at room temperature, then washed with automatic plate washer (5 x 300 $\mu$l/well with wash buffer, TBST). Detection antibody mouse anti-UCH-L1-HRP conjugated (made in-house, 50 $\mu$g/ml) in blocking buffer is then added to wells at 100$\mu$L/well and incubated for 1.5 h at room temperature, followed by washing. If amplification is needed, biotinyl-tyramide solution (Perkin Elmer Elast Amplification Kit) is added for 15 min at room temperature, washed then followed by 100 $\mu$l/well streptavidin-HRP (1:500) in PBS with 0.02% Tween-20 and 1% BSA for 30 min and then followed by washing. Lastly, the wells are developed with 100$\mu$l/well TMB substrate solution (Ultra-TMB ELISA, Pierce# 34028) with incubation times of 5-30 minutes. The signal is read at 652 nm with a 96-well spectrophotometer (Molecular Device Spectramax 190).

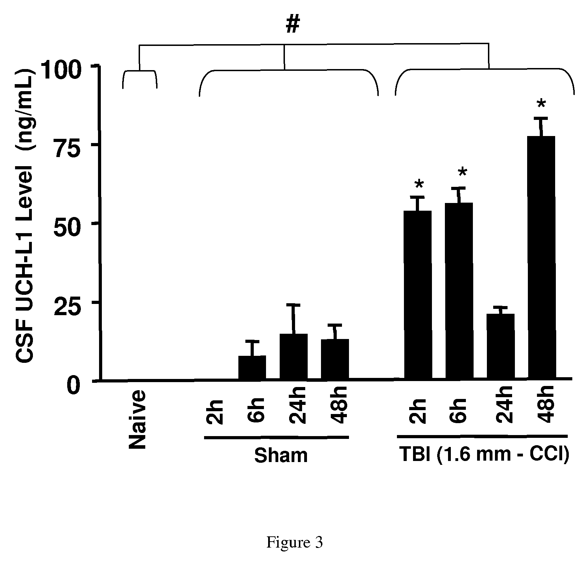

UCH-L1 levels of the TBI group (percussive injury) are significantly higher than the sham controls (p<0.01, ANOVA analysis) and the naive controls as measured by a swELISA demonstrating that UCHL1 is elevated early in CSF (2h after injury) then declines at around 24 h after injury before rising again 48 h after injury (FIG. 3).

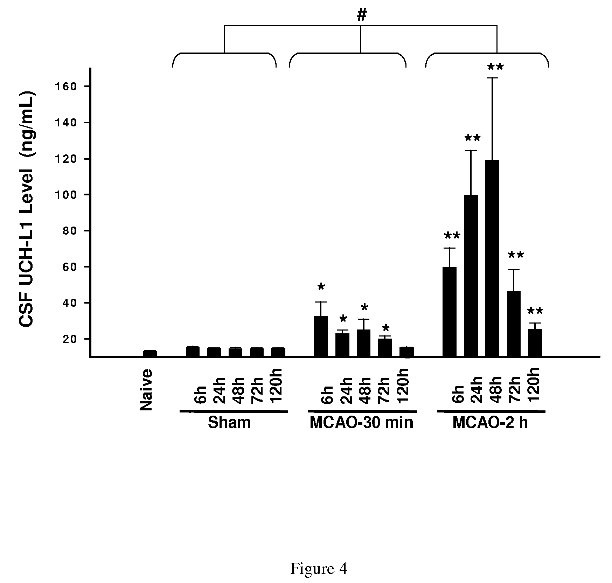

Following MCAO challenge the magnitude of UCHL1 in CSF is dramatically increased with severe (2h) challenge relative to a more mild challenge (30 min). (FIG. 4.) The more severe 2 h MCAO group UCHL1 protein levels are 2-5 fold higher than 30 min MCAO (p < 0.01, ANOVA analysis). UCHL1 protein levels for shams are virtually indistinguishable from naive controls.

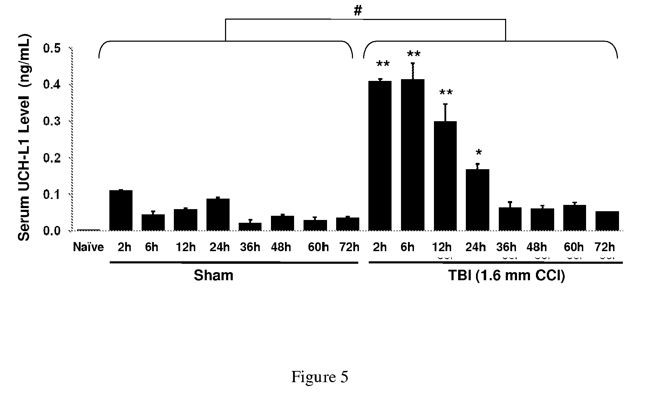

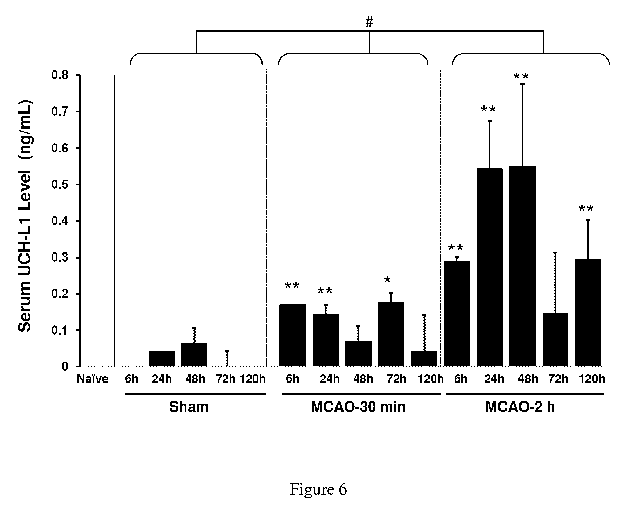

Similar results are obtained for UCHL1 in serum. Blood (3-4 ml) is collected at the end of each experimental period directly from the heart using syringe equipped with 21 gage needle placed in a polypropylene tube and allowed to stand for 45 min to 1 hour at room temperature to form clot. Tubes are centrifuged for 20 min at 3,000xg and the serum removed and analyzed by ELISA (FIGs. 5, 6.)

UCHL1 levels of the TBI group are significantly higher than the sham group (p < 0.001, ANOVA analysis) and for each time point tested 2 h through 24 h respective to the same sham time points (p < 0.005, Student's T-test). UCH-L1 is significantly elevated after injury as early as 2h in serum. Severe MCAO challenge produces increased serum UCHL1 relative to mild challenge. Both mild and severe challenge are statistically higher than sham treated animals indicating that serum detection of UCHL1 is a robust diagnostic and the levels are able to sufficiently distinguish mild from severe injury.

## Example 8:

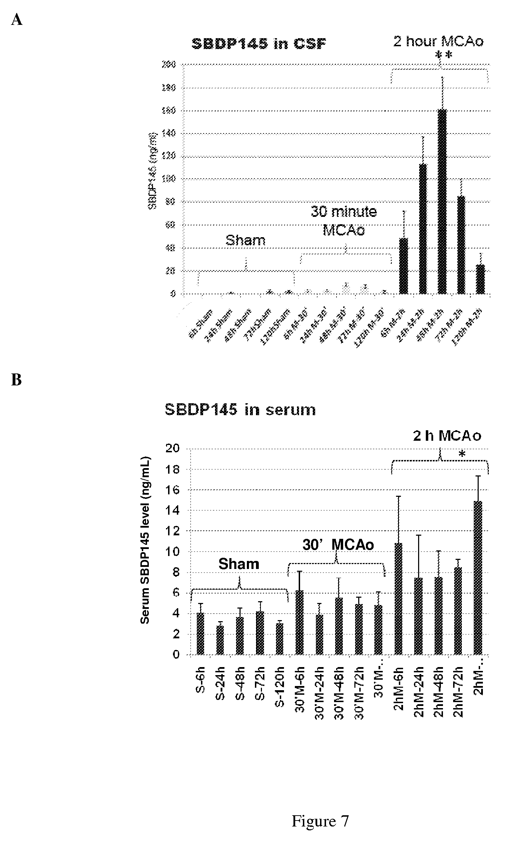

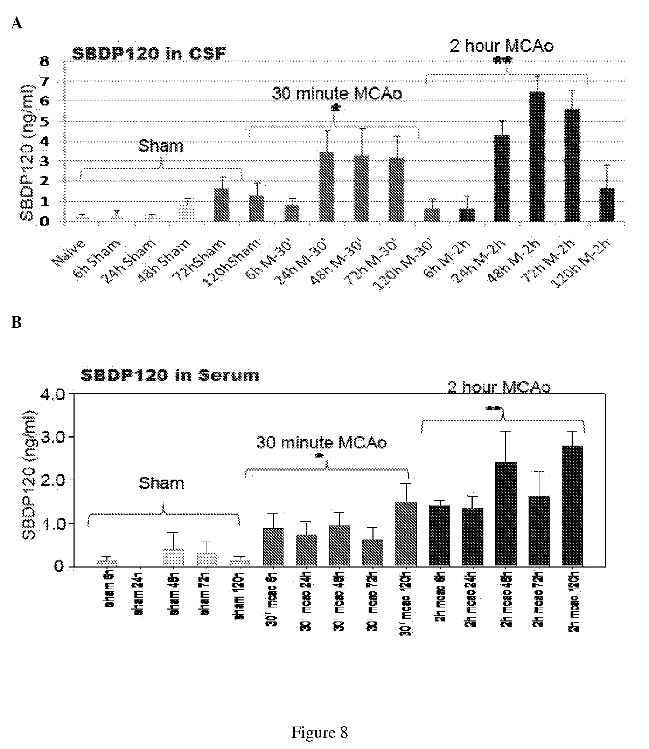

Spectrin breakdown products are analyzed following rat MCAO challenge by procedures similar to those described in U.S. Patent No. 7,291,710, the contents of which are incorporated herein by reference. FIG. 7 demonstrates that levels of SBDP145 in both serum and CSF are significantly (p<0.05) increased at all time points studied following severe (2hr) MCAO challenge relative to mild (30 min) challenge. Similarly, SBDP120 demonstrates significant elevations following severe MCAO challenge between 24 and 72 hours after injury in CSF (FIG. 8). However, levels of SBDP120 in serum are increased following severe challenge relative to mild challenge at all time points between 2 and 120 hours. In both CSF and Serum both mild and severe MCAO challenge produces increased SPBP120 and 140 relative to sham treated subjects.

## Example 9:

Microtubule Associated Protein 2 (MAP2) is assayed as a biomarker in both CSF and serum following mild (30 min) and severe (2 hr) MCAO challenge in subjects by ELISA or western blotting essentially as described in Examples 4 and 7. Antibodies to MAP2 (MAP-2 (E-12)) are obtained from Santa Cruz Biotechnology, Santa Cruz, CA. These antibodies are suitable for both ELISA and western blotting procedures and are crossreactive to murine and human MAP2. Levels of MAP2 are significantly (p<0.05) increases in subjects following mild MCAO challenge relative to naive animals in both CSF and Serum (FIG. 9). Similar to UCHL1 and SBDPs, severe challenge (2 hr) produces much higher levels of MAP2 in both samples than mild challenge (30 min).

## Example 10:

Dicyclomine inhibits production of SBDPs in CSF following TBI but has no effect on neuronal cell viability. The therapeutic dicyclomine is analyzed for its ability to alter levels of biomarkers in biological samples following challenge essentially as described by Cox, CD, et al., J Neurotrauma, 2008; 25(11):1355-65 the contents of which are incorporated herein by reference.

Eight TBI subjects without drug treatment are used in a time course study for SPDP immunohistochemistry (n=2/time point at 3, 12, 24, and 48 h post-TBI). Twenty four subjects are assigned to one of three treatment groups (n = 8 each): an injury group administered a saline vehicle (equal volume as drug injection); an injury group administered dicyclomine; and a third, sham-injury group to serve as an uninjured baseline from which to compare TBI-induced changes in SBDP levels. All procedures are performed in compliance with a protocol approved by the UC Davis Institutional Animal Care and Use Committee.

Subjects are administered CCI essentially as described in Example 2 and CSF is prepared as described in Example 4. Dicyclomine is dissolved in isotonic saline and administered intraperitoneally (i.p.) in a 5 mg/kg dose (volume 1.0 ml/kg) five minutes prior to induction of TBI. The vehicle-treatment group receives an equal volume i.p. injection of isotonic saline. Both groups receive injections at the same time points relative to the injury, with the identity of the drug or vehicle concealed to the investigators.

There are no significant differences between groups for TBI magnitude, initial body weight (at the time of surgery), or temperatures during surgery (Table 1). Percent body weight loss is significantly reduced (p < 0.01) in the dicyclomine-treated group compared to the saline-treated group (Table 1).

**Table 1**

| | Initial Body Weight(g) | % Body Weight Loss | TBI Magnitude (AIM) | Pre-TBI | | Post-TBI | |
|---|---|---|---|---|---|---|---|
| | | | | Body Temp (°C) | Brain Temp(°C) | Body Temp(°C) | BtamTeirp.CO |
| Sham | 3-4 +/- 0.007 | 3.8 +/- 209 | - | 37.0 +/- 0.220 | 361 +/- Q104 | 37.0 +/- 0.349 | 36.1 +/ 0.100 |
| Vehicle | 329 +/- 0.014 | 11.1 +/- 1.10 | 2.15+/- 0.009 | 369+/- 0336 | 35.9+/- 0.203 | 36.9+/ 0.319 SD | 36.0 +/0.238 |
| Dicyclomine | 315 +/- 0.016 | *7.84 +/-208 | 2.15 +/- 0.013 | 37.2 +/- 0330 | 360 +/-028 | 37.0 +/083 SD | 35.0 +/-000 |

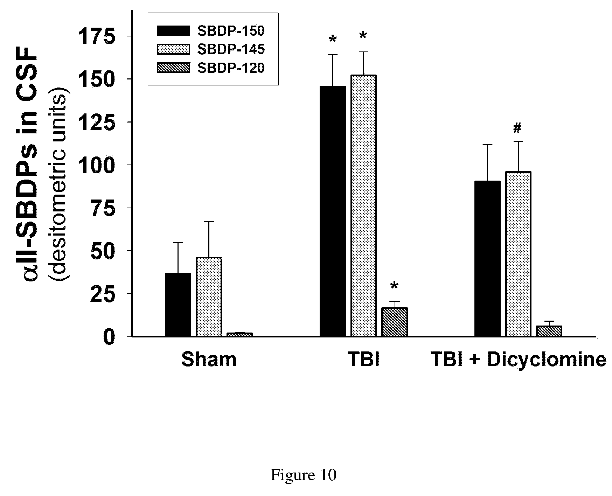

FIG. 10 demonstrates that SBDP levels are statistically increased following CCI. Mean $\alpha$-II SBDP levels measured in CSF are significantly elevated in both injury groups at 24 hours post-TBI compared with sham animals (p < 0.001). 145 kDa SBDP levels are significantly (p = 0.028) reduced in the dicyclomine treatment group compared to the vehicle treatment group.

Fluoro-Jade is used to determine whether the presence of dicyclomine affects cell death following CCI. Experimental procedures are performed as described by Cox, CD, et al. 2008. Briefly, at 24 hours ($\pm$ 1 hr) following the perfusion, brains are rinsed in 0.1M PB (5 minutes X 2), cryoprotected in 10% and 30% solutions of sucrose in 0.1M PB for 1 hour and 48 hours, respectively, and stored in the 30% sucrose/PB solution at -80 °C. The brains are blocked and sectioned caudorostrally in 45 $\mu$m increments from Bregma - 1.9 mm to Bregma - 4.15 mm using a sliding microtome (American Optical Corp., model 860). The tissue sections are then mounted onto slides using distilled water and allowed to dry. For the Fluoro-Jade staining procedure (Schmued et al., 1997), the sections are rehydrated using successive 5 minute rinses in 100%, 75%, 50%, and 25% ethanol followed by 3 minutes in $dH_2O$, placed in 0.06% $KMNO_4$ for 15 minutes followed by 2 minutes in $dH_2O$, and then stained in 0.0006% Fluoro-Jade B solution in 0.1% acetic acid for 30 minutes. The slides are air-dried overnight, immersed in xylene, and coverslipped with DPX.

The total number of Fluoro-Jade stained cells within the CA2/CA3 region of the dorsal hippocampus for each subject is estimated using the optical fractionator technique (West et al., 1991) with a computer-based system (Stereologer version 1.3, Systems Planning and Analysis, Inc., Alexandria, VA). The border of the dorsal CA2/3 pyramidal cell layer within each section was outlined using a 2X objective, and the cells are then manually counted at the 40X magnification. For the cortical cell counts, a site of highly localized Fluoro-Jade positivity observed in the cortex adjacent to the site of impact serves as the ROI: the region was outlined at the 2X magnification and cells were quantified at 40X as with the hippocampal region. A numerical estimate of the total number of cells in each region of interest for each subject is calculated by the software using the equation:

$$N_{obj} = (N)(1/SSF)(1/ASF)(1/TSF)$$

Where N represents the sum of all objects counted for the subject, SSF the section sampling fraction, ASF the area sampling fraction, and TSF the thickness sampling fraction.

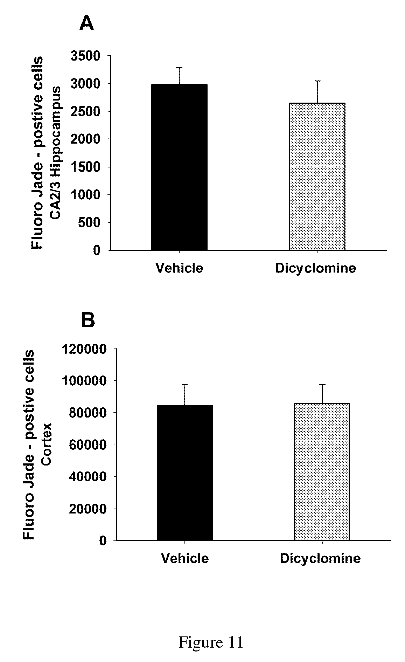

The mean numbers of Fluoro-Jade positive neurons observed in the hippocampal ROIs at 24 hours post-TBI were 2982 and 2640 for the vehicle and dicyclomine treated groups, respectively (FIG. 11A). Mean numbers for the cortical ROIs were 84392 and 84452 for vehicle and dicyclomine-treated groups, respectively (FIG. 11B). T-test revealed no significant difference between the treatment groups in either region. These results suggest that dicyclomine may not affect cell death, but is expected to correlate with improved behavioral characteristics for which the biomarkers may indicate an appreciable therapeutic outcome.

Patents and publications mentioned in the specification are indicative of the levels of those skilled in the art to which the invention pertains. These patents and publications are incorporated herein by reference to the same extent as if each individual application or publication was specifically and individually expressed explicitly in detail herein.

The foregoing description is illustrative of particular embodiments of the invention, but is not meant to be a limitation upon the practice thereof. The following claims, including all equivalents thereof, are intended to define the scope of the invention.

The following references are each incorporated herein by reference as if the contents of each reference were fully and explicitly included.

Reference List

US. Patent Nos. 7,291,710 and 6,187,756.

US Publication Nos. 2008/0254482 and 2007/0105114.

Amaral, D. G., Witter, M. P.(1995) Hippocampal formation. In: G. Paxinos, Ed., The Rat Nervous System. vol. Academic Press, San Diego, pp. 443-493.

Auerbach, J. M., Segal, M. (1996). Muscarinic receptors mediating depression and long-term potentiation in rat hippocampus. J Physiol. 492 (Pt 2), 479-93.

Bornstein, M. B. (1946). Presence and action of acetylcholine in experimental brain trauma. Journal of Neurophysiology. 9, 349-366.

Burgard, E. C., Sarvey, J. M. (1990). Muscarinic receptor activation facilitates the induction of long-term potentiation (LTP) in the rat dentate gyrus. Neurosci Lett. 116, 34-9.

Czogalla, A., Sikorski, A. F. (2005). Spectrin and calpain: a 'target' and a 'sniper' in the pathology of neuronal cells. Cell Mol Life Sci. 62, 1913-24.

DeAngelis, M. M., Hayes, R. L., Lyeth, B. G. (1994). Traumatic brain injury causes a decrease in M2 muscarinic cholinergic receptor binding in the rat brain. Brain Res. 653, 39-44.

Delahunty, T. M. (1992). Mild traumatic brain injury enhances muscarinic receptor-linked inositol phosphate production in rat hippocampus. Brain Res. 594, 307-10.

Delahunty, T. M., Jiang, J. Y., Black, R. T., Lyeth, B. G. (1995a). Differential modulation of carbachol and trans-ACPD-stimulated phosphoinositide turnover following traumatic brain injury. Neurochem Res. 20, 405-11.

Delahunty, T. M., Jiang, J. Y., Gong, Q. Z., Black, R. T., Lyeth, B. G. (1995b). Differential consequences of lateral and central fluid percussion brain injury on receptor coupling in rat hippocampus. J Neurotrauma. 12, 1045-57.

Dhillon, H. S., Carbary, T., Dose, J., Dempsey, R. J., Prasad, M. R. (1995). Activation of phosphatidylinositol bisphosphate signal transduction pathway after experimental brain injury: A lipid study. Brain Research. 698, 100-106.

Dixon, C. E., Lyeth, B. G., Povlishock, J. T., Findling, R. L., Hamm, R. J., Marmarou, A., Young, H. F., Hayes, R. L. (1987). A fluid percussion model of experimental brain injury in the rat. J Neurosurg. 67, 110-9.

Dixon, C. E., Markgraf, C. G., Angileri, F., Pike, B. R., Wolfson, B., Newcomb, J. K., Bismar, M. M., Blanco, A. J., Clifton, G. L., Hayes, R. L. (1998). Protective effects of moderate hypothermia on behavioral deficits but not necrotic cavitation following cortical impact injury in the rat. Journal of Neurotrauma. 15, 95-103.

Doods, H. N., Mathy, M. J., Davidesko, D., van Charldorp, K. J., de Jonge, A., van Zwieten, P. A. (1987). Selectivity of muscarinic antagonists in radioligand and in vivo experiments for the putative M1, M2 and M3 receptors. Journal of Pharmacology and Experimental Therapeutics. 242, 257-262.

Farkas, O., Lifshitz, J., Povlishock, J. T. (2006). Mechanoporation induced by diffuse traumatic brain injury: an irreversible or reversible response to injury? J Neurosci. 26, 3130-40.

Fineman, I., Hovda, D. A., Smith, M., Yoshino, A., Becker, D. P. (1993). Concussive brain injury is associated with a prolonged accumulation of calcium: a 45Ca autoradiographic study. Brain Res. 624, 94-102.

Fleminger, S., Oliver, D. L., Lovestone, S., Rabe-Hesketh, S., Giora, A. (2003). Head injury as a risk factor for Alzheimer's disease: the evidence 10 years on; a partial replication. J Neurol Neurosurg Psychiatry. 74, 857-62.

Gallo, M. P., Alloatti, G., Eva, C., Oberto, A., Levi, R. C. (1993). M1 muscarinic receptors increase calcium current and phosphoinositide turnover in guinea-pig ventricular cardiocytes. J Physiol. 471, 41-60.

Gorman, L. K., Fu, K., Hovda, D. A., Becker, D. P., Katayama, Y. (1989). Analysis of acetylcholine release following concussive brain injury in the rat. J Neurotrauma. 6, 203.

Haber, B., Grossman, R. G.(1980) Acetylcholine metabolism in intracranial and lumbar cerebrospinal fluid and in blood. In: J. H. Wood, Ed., Neurobiology of Cerebrospinal Fluid. vol. 1. Plenum, New York, pp. 345-350.

Hallam, T. M., Floyd, C. L., Folkerts, M. M., Lee, L. L., Gong, Q. Z., Lyeth, B. G., Muizelaar, J. P., Bermnan, R. F. (2004). Comparison of behavioral deficits and acute neuronal degeneration in rat lateral fluid percussion and weight-drop brain injury models. J Neurotrauma. 21, 521-39.

Hamm, R. J., Dixon, C. E., Gbadebo, D. M., Singha, A. K., Jenkins, L. W., Lyeth, B. G., Hayes, R. L. (1992). Cognitive deficits following traumatic brain injury produced by controlled cortical impact. J Neurotrauma. 9, 11-20.

Heppner, F., Diemath, H. E. (1958). [Clinical experiences with anticholinergic treatment of closed intracranial trauma.]. Monatsschrift fur Unfallheilkunde und Versicherungsmedizin. 61, 257-65.

Jenkner, F. L., Lechner, H. (1954). [The effects of scopolamine on the electroencephalogram in a new closed brain injury.]. Der Nervenarzt. 25, 77-8.

Jenkner, F. L., Lechner, H. (1955). The effect of diparcol on the electroencephalogram in the normal subject and in those with cerebral trauma. Electroencephalogr Clin Neurophysiol. 7,303-5.

Jiang, J. Y., Lyeth, B. G., Delahunty, T. M., Phillips, L. L., Hamm, R. J. (1994). Muscarinic cholinergic receptor binding in rat brain at 15 days following traumatic brain injury. Brain Res. 651, 123-8.

Jiang, Z. W., Gong, Q. Z., Di, X., Zhu, J., Lyeth, B. G. (2000). Dicyclomine, an M1 muscarinic antagonist, reduces infarct volume in a rat subdural hematoma model. Brain Res. 852, 37-44.

Johnson, G. V., Jope, R. S., Binder, L. I. (1989). Proteolysis of tau by calpain. Biochem Biophys Res Commun. 163, 1505-11.

Kampfl, A., Posmantur, R. M., Zhao, X., Schmutzhard, E., Clifton, G. L., Hayes, R. L. (1997). Mechanisms of calpain proteolysis following traumatic brain injury: Implications for pathology and therapy: A review and update. J.Neurotrauma. 14, 121-134.

Kumagae, Y., Matsui, Y. (1991). Output, tissue levels, and synthesis of acetylcholine during and after transient forebrain ischemia in the rat. Journal of Neurochemistry. 56, 1169-1173.

Langlois, J. A., Rutland-Brown, W., Thomas, K. E. (2006). Traumatic Brain Injury in the United States: Emergency Department Visits, Hospitalization, and Deaths, vol. Atlanta (GA) Centers for Disease Control and Prevention, National Center for Injury Prevention and Control

Lewis, S. B., Velat, G. J., Miralia, L., Papa, L., Aikman, J. M., Wolper, R. A., Firment, C. S., Liu, M. C., Pineda, J. A., Wang, K. K., Hayes, R. L. (2007). Alpha-II spectrin breakdown products in aneurysmal subarachnoid hemorrhage: a novel biomarker of proteolytic injury. J Neurosurg. 107, 792-6.

Liu, X., Van Vleet, T., Schnellmann, R. G. (2004). The role of calpain in oncotic cell death. Annu Rev Pharmacol Toxicol. 44, 349-70.

Lye, T. C., Shores, E. A. (2000). Traumatic brain injury as a risk factor for Alzheimer's disease: a review. Neuropsychology review. 10, 115-29.

Lyeth, B. G., Dixon, C. E., Hamm, R. J., Jenkins, L. W., Young, H. F., Stonnington, H. H., Hayes, R. L. (1988a). Effects of anticholinergic treatment on transient behavioral suppression and physiological responses following concussive brain injury to the rat. Brain Res. 448, 88-97.

Lyeth, B. G., Dixon, C. E., Jenkins, L. W., Hamm, R. J., Alberico, A., Young, H. F., Stonnington, H. H., Hayes, R. L. (1988b). Effects of scopolamine treatment on long-term behavioral deficits following concussive brain injury to the rat. Brain Res. 452, 39-48.

Lyeth, B. G., Gong, Q. Z., Dhillon, H. S., Prasad, M. R. (1996). Effects of muscarinic receptor antagonism on the phosphatidylinositol bisphosphate signal transduction pathway after experimental brain injury. Brain Res. 742, 63-70.

Lyeth, B. G., Hayes, R. L. (1992). Cholinergic and opioid mediation of traumatic brain injury. J Neurotrauma. 9 Suppl 2, S463-74.

Lyeth, B. G., Jenkins, L. W., Hamm, R. J., Dixon, C. E., Phillips, L. L., Clifton, G. L., Young, H. F., Hayes, R. L. (1990). Prolonged memory impairment in the absence of hippocampal cell death following traumatic brain injury in the rat. Brain Res. 526, 249-58.

Lyeth, B. G., Jiang, J. Y., Delahunty, T. M., Phillips, L. L., Hamm, R. J. (1994). Muscarinic cholinergic receptor binding in rat brain following traumatic brain injury. Brain Res. 640, 240-5.

Lyeth, B. G., Jiang, J. Y., Robinson, S. E., Guo, H., Jenkins, L. W. (1993a). Hypothermia blunts acetylcholine increase in CSF of traumatically brain injured rats. Mol Chem Neuropathol. 18, 247-56.

Lyeth, B. G., Liu, S., Hamm, R. J. (1993b). Combined scopolamine and morphine treatment of traumatic brain injury in the rat. Brain Res. 617, 69-75.

Mandelkow, E. M., Mandelkow, E. (1998). Tau in Alzheimer's disease. Trends in cell biology. 8,425-7.

Max, W., MacKenzie, E. J., Rice, D. P. (1991). Head injuries: costs and consequences. J Head Trauma Rehabil 6, 76-91.

McAllister, T. W. (1992). Neuropsychiatric sequelae of head injuries. The Psychiatric clinics of North America. 15, 395-413.

Mcintosh, T. K., Vink, R., Noble, L., Yamakami, I., Fernyak, S., Soares, H., Faden, A. I. (1989). Traumatic brain injury in the rat: characterization of a lateral fluid-percussion model. Neuroscience. 28, 233-244.

Miyazaki, S., Katayama, Y., Lyeth, B. G., Jenkins, L. W., DeWitt, D. S., Goldberg, S. J., Newlon, P. G., Hayes, R. L. (1992). Enduring suppression of hippocampal long-term potentiation following traumatic brain injury in rat. Brain Res. 585, 335-9.

Olney, J. W., de Gubareff, T., Labruyere, J. (1983). Seizure-related brain damage induced by cholinergic agents. Nature. 301, 520-522.

Pike, B. R., Flint, J., Dave, J. R., Lu, X. C., Wang, K. K., Tortella, F. C., Hayes, R. L. (2004). Accumulation of calpain and caspase-3 proteolytic fragments of brain-derived alphaII-spectrin in cerebral spinal fluid after middle cerebral artery occlusion in rats. J Cereb Blood Flow Metab. 24, 98-106.

Pike, B. R., Flint, J., Dutta, S., Johnson, E., Wang, K. K., Hayes, R. L. (2001). Accumulation of non-erythroid alpha II-spectrin and calpain-cleaved alpha II-spectrin breakdown products in cerebrospinal fluid after traumatic brain injury in rats. J Neurochem. 78, 1297-306.

Pineda, J. A., Lewis, S. B., Valadka, A. B., Papa, L., Hannay, H. J., Heaton, S. C., Demery, J. A., Liu, M. C., Aikman, J. M., Akle, V., Brophy, G. M., Tepas, J. J., Wang, K. K., Robertson, C. S., Hayes, R. L. (2007). Clinical significance of alphaII-spectrin breakdown products in cerebrospinal fluid after severe traumatic brain injury. J Neurotrauma. 24, 354-66.

Prasad, M. R., Dhillon, H. S., Carbary, T., Dempsey, R. J., Scheff, S. W. (1994). Enhanced phosphodiestric breakdown of phosphatidylinositol bisphosphate after experimental brain injury. J Neurochem. 63, 773-776.

Reeves, T. M., Lyeth, B. G., Povlishock, J. T. (1995). Long-term potentiation deficits and excitability changes following traumatic brain injury. Exp Brain Res. 106, 248-56.

Riederer, B. M., Zagon, I. S., Goodman, S. R. (1986). Brain spectrin(240/235) and brain spectrin(240/235E): two distinct spectrin subtypes with different locations within mammalian neural cells. J Cell Biol. 102, 2088-97.

Ringger, N. C., O'Steen, B. E., Brabham, J. G., Silver, X., Pineda, J., Wang, K. K., Hayes, R. L., Papa, L. (2004). A novel marker for traumatic brain injury: CSF alphaII-spectrin breakdown product levels. J Neurotrauma. 21, 1443-56.

Robinson, S. E., Foxx, S. D., Posner, M. G., Martin, R. M., Davis, T. R., Guo, H., Enters, E. K. (1990). The effect of M1 muscarinic blockade on behavior and physiological responses following traumatic brain injury in the rat. Brain Res. 511, 141-148.

Sachs, E., Jr. (1957). Acetylcholine and serotonin in the spinal fluid. J Neurosurg. 14, 22-7.

Saido, T. C., Sorimachi, H., Suzuki, K. (1994). Calpain: new perspectives in molecular diversity and physiological-pathological involvement. Faseb J. 8, 814-22.

Schmued, L. C., Albertson, C., Slikker, W. J. (1997). Fluoro-Jade: a novel fluorochrome for the sensitive and reliable histochemical localization of neuronal degeneration. Brain Research. 751, 37-46.

Schmued, L. C., Hopkins, K. J. (2000). Fluoro-Jade: novel fluorochromes for detecting toxicant-induced neuronal degeneration. Toxicologic Pathology. 28, 91-99.

Sharma, V. K., Colecraft, H. M., Wang, D. X., Levey, A. I., Grigorenko, E. V., Yeh, H. H., Sheu, S. S. (1996). Molecular and functional identification of m1 muscarinic acetylcholine receptors in rat ventricular myocytes. Circ Res. 79, 86-93.

Thompson, H. J., Lifshitz, J., Marklund, N., Grady, M. S., Graham, D. I., Hovda, D. A., McIntosh, T. K. (2005). Lateral fluid percussion brain injury: a 15-year review and evaluation. J Neurotrauma. 22, 42-75.

Tower, D. B., McEachern, D. (1949). Acetylcholine and neuronal activity; cholinesterase patterns and acetylcholine in the cerebrospinal fluids of patients with craniocerebral trauma. Canadian journal of research. 27, 105-19.

Turski, W. A., Czuczwar, S. J., Kleinrok, Z., Turski, L. (1983). Cholinomimetics produce seizures and brain damage in rats. Experientia. 39, 1408-11.

Wang, K. K. (2000). Calpain and caspase: can you tell the difference? Trends Neurosci. 23, 20-6.

Wang, K. K., Ottens, A. K., Liu, M. C., Lewis, S. B., Meegan, C., Oli, M. W., Tortella, F. C., Hayes, R. L. (2005). Proteomic identification of biomarkers of traumatic brain injury. Expert review of proteomics. 2, 603-14.

Wang, K. K. W.(2002) Calpain and caspase in ischemic and traumtic brain injury. In: R. Lin, Ed., New Concepts in Cerebral Ischemia. vol. CRC Press, New York, pp. 181-200.

Ward Jr., A. (1950). Atropine in the treatment of closed head injury. J Neurosurg. 7, 398-402.

Wei, E. P., Lamb, R. G., Kontos, H. A. (1982). Increased phospholipase C activity after experimental brain injury. J Neurosurg. 56, 695-8.

West, M. J., Slomianka, L., Gundersen, H. J. (1991). Unbiased stereological estimation of the total number of neurons in the subdivisions of the rat hippocampus using the optical fractionator. Anatomical Record. 231, 482-497.

Berger RP, Adelson PD, Pierce MC, Dulani T, Cassidy LD, Kochanek PM (2005) Serum neuron-specific enolase, S100B, and myelin basic protein concentrations after inflicted and noninflicted traumatic brain injury in children. J Neurosurg 103:61-68

Choi DW (2002) Exploratory clinical testing of neuroscience drugs. Nat Neurosci Suppl: 1023-1025.

Choi SC, Bullock R. (2001) Design and statistical issues in multicenter trials of severe head injury. Neurol Res 23:190-192 Review.

Coleman MP, Ribchester RR (2004) Programmed axon death, synaptic dysfunction and the ubiquitin proteasome system. Curr Drug Targets CNS Neurol Disord 3:227-238

Cooper EH (1994) Neuron-specific enolase. Int J Biol Markers 9:205-210

Dambinova SA, Khounteev GA, Izykenova GA, Zavolokov IG, Ilyukhina AY, Skoromets AA (2003) Blood test detecting autoantibodies to N-methyl-D-aspartate neuroreceptors for evaluation of patients with transient ischemic attack and stroke. Clin Chem 49:1752-1762

Doran JF, Jackson P, Kynoch PA, Thompson RJ (1983) Isolation of PGP 9.5, a new human neurone-specific protein detected by high-resolution two-dimensional electrophoresis. J Neurochem 40:1542-1547

Ge P, Luo Y, Liu CL, Hu B (2007) Protein aggregation and proteasome dysfunction after brain ischemia. Stroke 38:3230-3236

Gong B, Leznik E (2007) The role of ubiquitin C-terminal hydrolase L1 in neurodegenerative disorders. J Cereb Blood Flow Metab 20:365-370

Hu BR, Janelidze S, Ginsberg MD, Busto R, Perez-Pinzon M, Sick TJ, Siesjo BK, Liu CL (2001) Protein aggregation after focal brain ischemia and reperfusion. J Cereb Blood Flow Metab 21:865-875

Jackson P, Thompson RJ (1981) The demonstration of new human brain-specific proteins by high-resolution two-dimensional polyacrylamide gel electrophoresis. J Neurol Sci 49: 429-438

Johnsson P, Blomquist S, Lührs C, Malmkvist G, Alling C, Solem JO, Stahl E (2000) Neuron-specific enolase increases in plasma during and immediately after extracorporeal circulation. Ann Thorac Surg 69:750-4

Johnston SC, Riddle SM, Cohen RE, Hill CP (1999) Structural basis for the specificity of ubiquitin C-terminal hydrolases. EMBO J 18:3877-3887

Keller JN, Huang FF, Zhu H, Yu J, Ho YS, Kindy TS (2000) Oxidative stress-associated impairment of proteasome activity during ischemia-reperfusion injury. J Cereb Blood Flow Metab 20:1467-1473

Kobeissy FH, Ottens AK, Zhang ZQ, Dave JR, Tortella FC, Hayes RL Wang KKW (2006) Novel differential neuroproteomics analysis of traumatic brain injury in rats. Mol Cell Proteomics 5:1887-1898.

Kwon J, Mochida K, Wang YL, Sekiguchi S, Sankai T, Aoki S, Ogura A, Yoshikawa Y, Wada K (2005) Ubiquitin C-terminal hydrolase L-1 is essential for the early apoptotic wave of germinal cells and for sperm quality control during spermatogenesis. Biol Reprod 73:29-35.

Langlois JA, Rutland-Brown W, Thomas KE (2004) Traumatic brain injury in the United States: Emergency department visits, hospitalizations, and deaths. National Center for Injury Prevention and Control, Centers for Disease Control and Prevention.

Larsen CN, Price JS, Wilkinson KD (1996) Substrate binding and catalysis by ubiquitin C-terminal hydrolases: identification of two active site residues. Biochemistry 35:6735-6744

Larsen CN, Krantz BA, Wilkinson KD (1998). Substrate specificity of deubiquitinating enzymes: ubiquitin C-terminal hydrolases. Biochemistry 37: 3358-3368

Laser H, Mack TG, Wagner D, Coleman MP (2003) Proteasome inhibition arrests neurite outgrowth and causes "dying-back" degeneration in primary culture. J Neurosci Res 74:906-916

Lincoln S, Vaughan J, Wood N, Baker M, Adamson J, Gwinn-Hardy K, Lynch T, Hardy J, Farrer M (1999) Low frequency of pathogenic mutations in the ubiquitin carboxy-terminal hydrolase gene in familial Parkinson's disease. Neuroreport 10:427-429

Missler U, Wiesmann M, Friedrich C, Kaps M (1997) S-100 protein and neuron-specific enolase concentrations in blood as indicators of infarction volume and prognosis in acute ischemic stroke. Stroke 28:1956-1960

Pelinka LE, Szalay L, Jafarmadar M, Schmidhammer R, Redl H, Bahrami S (2003) Circulating S100B is increased after bilateral femur fracture without brain injury in the rat. Br J Anaesth. 91:595-7

Pelinka LE, Kroepfl A, Schmidhammer R, Krenn M, Buchinger W, Redl H, Raabe A (2004a) Glial fibrillary acidic protein in serum after traumatic brain injury and multiple trauma. J Trauma 57:1006-1012

Pelinka LE, Kroepfl A, Leixnering M, Buchinger W, Raabe A, Redl H (2004b) GFAP versus S100B in serum after traumatic brain injury: relationship to brain damage and outcome. J Neurotrauma 21:1553-1561

Pelinka LE, Harada N, Szalay L, Jafarmadar M, Redl H, Bahrami S (2004c) Release of S100B differs during ischemia and reperfusion of the liver, the gut, and the kidney in rats. Shock. 21:72-6

Pike BR, Flint J, Dave JR, Lu XC, Wang KKW, Tortella FC, Hayes RL (2003) Accumulation of calpain and caspase-3 proteolytic fragments of brain-derived $\alpha$II-spectrin in CSF after middle cerebral artery occlusion in rats. J Cereb Blood Flow Metab 24: 98-106

Pike BR, Flint J, Johnson E, Glenn CC, Dutta S, Wang KKW, Hayes RL (2001) Accumulation of Calpain-Cleaved Non-Erythroid $\alpha$II-Spectrin in Cerebrospinal Fluid after Traumatic Brain Injury in Rats. J Neurochem 78:1297-1306.

Raabe A, Grolms C, Seifert V (1999) Serum markers of brain damage and outcome prediction in patients after severe head injury. Br J Neurosurg 13:56-59

Romner B, Ingebrigtsen T, Kongstad P, Borgesen SE (2000) Traumatic brain damage: serum S-100 protein measurements related to neuroradiological findings. J Neurotrauma 17:641-647

Ross SA, Cunningham RT, Johnston CF, Rowlands BJ (1996) Neuron-specific enolase as an aid to outcome prediction in head injury. Br J Neurosurg 10:471-476

Saigoh K, Wang YL, Suh JG, Yamanishi T, Sakai Y, Kiyosawa H, Harada T, Ichihara N, Wakana S, Kikuchi T, Wada K (1999) Intragenic deletion in the gene encoding ubiquitin carboxy-terminal hydrolase in gad mice. Nat Genet 23:47-51

Shaw GJ, Jauch EC, Zemlan FP (2002) Serum cleaved tau protein levels and clinical outcome in adult patients with closed head injury. Ann Emerg Med 39:254-257

Thompson RJ, Doran JF, Jackson P, Dhillon AP, Rode J (1983) PGP 9.5--a new marker for vertebrate neurons and neuroendocrine cells. Brain Res 278:224-228.

Tongaonkar P, Chen L, Lambertson D, Ko B, Madura K (2000) Evidence for an interaction between ubiquitin-conjugating enzymes and the 26S proteasome. Mol Cell Biol 20: 4691-4698

Vos PE, Lamers KJ, Hendriks JC, van Haaren M, Beems T, Zimmerman C, van Geel W, de Reus H, Biert J, Verbeek MM (2004) Glial and neuronal proteins in serum predict outcome after severe traumatic brain injury. Neurology 62:1303-1310

Wang KKW, Ottens A, Liu MC, Lewis SB, Meegan C, Oli MW, Tortella FC, Hayes RL (2005) Proteomic identification of biomarkers of traumatic brain injury. Expert Reviews Proteomics 2:603-614

Wilkinson KD, Deshpande S, Larsen CN (1992) Comparisons of neuronal (PGP9.5) and non-neuronal ubiquitin C-terminal hydrolases. Biochem Soc Trans 20: 631-637

Wilkinson KD (1995) Roles of ubiquitinylation in proteolysis and cellular regulation. Ann Rev Nutr 15: 161-189

Wilkinson KD (1997) Regulation of ubiquitin-dependent processes by deubiquitinating enzymes. FASEB J 11:1245-1256

Yamazaki Y, Yada K, Morii S, Kitahara T, Ohwada T (1995) Diagnostic significance of serum neuron-specific enolase and myelin basic protein assay in patients with acute head injury. Surg Neurol 43:267-270

Zemlan FP, Jauch EC, Mulchahey JJ, Gabbita SP, Rosenberg WS, Speciale SG, Zuccarello M. (2002) C-tau biomarker of neuronal damage in severe brain injured patients: association with elevated intracranial pressure and clinical outcome. Brain Res 947:131-139

**Claims**

1. A therapeutic for use in the treatment of a neurological condition, wherein wherein said therapeutic is selected from the group comprising a muscarinic receptor antagonist, milameline, N-methyl-4-piperidinyl benzylate NMP, pilocarpine, pirenzepine, acetylcholine, methacholine, carbachol, bethanechol, muscarine, oxotremorine M, oxotremorine, thapsigargin, calcium channel blockers or agonists, nicotine, xanomeline, BuTAC, clozapine, olanzapine, cevimeline, aceclidine, arecoline, tolterodine, rociverine, IQNP, indole alkaloids, himbacine, cyclostellettamines and derivatives thereof, pro-drugs thereof, and combinations thereof wherein the subject to which the therapeutic is to be administered has been previously diagnosed for the neurological condition or possibility of a neurological condition by a process for the diagnosis of a neurological condition or possibility of a neurological condition in a subject which comprises

   i) determining the amount of a biomarker in a biological sample, and
   ii) determining the ratio of the quantity of the biomarker to a measured or previously established baseline level of the same biomarker in a subject known to be free of a neurological condition,

   wherein the biomarker is a selected from the group consisting of a UCHL1, a spectrin breakdown product, MAP2, neuronal degeneration, ubiquitin carboxyl-terminal esterase, a neuronally-localized intracellular protein, MAP-tau, C-tau, poly(ADP-ribose) polymerase or collapsing response mediator and
   wherein the biological sample is cerebro spinal fluid (CSF) or serum.

2. The therapeutic for use according to claim 1 wherein said neurological condition is selected from the group comprising: brain injury, or multiple-organ injury, or combinations thereof.

3. The therapeutic for use according to claim 1 wherein said biomarker ratio is greater than 2 or less than 0,5.

4. The therapeutic for use according to claim 1 wherein said therapeutic is a direct therapeutic or an indirect therapeutic.

5. The therapeutic for use of claim 1, wherein the neuronally-localized intracellular protein is a ubiquitin carboxyl-terminal hydrolase.

6. A therapeutic for use according to claim 5, wherein the therapeutic is a muscarinic receptor antagonist.

7. The therapeutic for use according to claim 6 wherein said muscarinic receptor antagonist is dicyclomine or scopolamine.

8. The therapeutic for use according to claim 6 wherein said muscarinic receptor antagonist is dicyclomine.

9. The therapeutic for use according to claims 7 or 8 wherein said neurological condition is selected from the group comprising: percussive brain injury, ischemic stroke; and multiple organ injury.

Figure 1

**A**

Sham

MCAO-30 min

| 6h | 24h | 2D | 3D | 5D | | 6h | 24h | 2D | 3D | 5D |

**B**

UCH-L1 level in rat CSF
(arbituary densitometric unit)

MCAO-30 min

Figure 3

Figure 4

Figure 5

Figure 6

A

B

Figure 7

A

B

Figure 8

Figure 10

**A**

**B**

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61063515 B
- US 61055737 B
- US 61085623 B
- US 500000 A
- US 7291710 B
- US 7396654 B
- US 5475096 A
- US 5670637 A
- US 5696249 A
- US 5270163 A
- US 5707796 A
- US 5595877 A
- US 5660985 A
- US 5567588 A
- US 5683867 A
- US 5637459 A
- US 6011020 A
- US 6187756 B
- US 20080254482 A
- US 20070105114 A

**Non-patent literature cited in the description**

- **KOBEISSY FH et al.** *Mol Cell Proteomics,* 2006, vol. 5, 1887-1898
- **COX, CD et al.** *J Neurotrauma,* 2008, vol. 25 (11), 1355-65
- **KOBEISSY, FH et al.** *Molecular & Cellular Proteomics,* 2006, vol. 5, 1887-1898
- **F. MEYER.** Atlas of Neurosurgery: Basic Approaches to Cranial and Vascular Procedures. Churchill Livingstone, 1999
- **DAVID G. T. THOMAS.** Stereotactic and Image Directed Surgery of Brain Tumors. WB Saunders Co, 1993
- **L. N. SEKHAR ; E. DE OLIVEIRA.** Cranial Microsurgery: Approaches and Techniques. Thieme Medical Publishing, 1999
- **BELAY et al.** *Arch. Neurol.,* 2001, vol. 58, 1673-1678
- **SEIJO et al.** *J. Clin. Microbiol.,* 2000, vol. 38, 3892-3895
- **LEE et al.** *Pharmacological Research,* 2006, vol. 23, 312-328
- Molecular Cloning: A Laboratory Manual. Cold Spring Harbor Laboratory Press, 1989
- Current Protocols in Molecular Biology. Greene Publishing and Wiley-Interscience, 1992
- Current Protocols in Immunology. John Wiley & Sons, 1991
- Methods of Immunological Analysis. John Wiley & Sons, 1992
- Guide for the Care and Use of Laboratory Animals. NRC Publication, 1996
- Hippocampal formation. **AMARAL, D. G. ; WITTER, M. P.** The Rat Nervous System. Academic Press, 1995, 443-493
- **AUERBACH, J. M. ; SEGAL, M.** Muscarinic receptors mediating depression and long-term potentiation in rat hippocampus. *J Physiol.,* 1996, vol. 492, 479-93
- **BORNSTEIN, M. B.** Presence and action of acetylcholine in experimental brain trauma. *Journal of Neurophysiology,* 1946, vol. 9, 349-366
- **BURGARD, E. C. ; SARVEY, J. M.** Muscarinic receptor activation facilitates the induction of long-term potentiation (LTP) in the rat dentate gyrus. *Neurosci Lett.,* 1990, vol. 116, 34-9
- **CZOGALLA, A. ; SIKORSKI, A. F.** Spectrin and calpain: a 'target' and a 'sniper' in the pathology of neuronal cells. *Cell Mol Life Sci.,* 2005, vol. 62, 1913-24
- **DEANGELIS, M. M. ; HAYES, R. L. ; LYETH, B. G.** Traumatic brain injury causes a decrease in M2 muscarinic cholinergic receptor binding in the rat brain. *Brain Res.,* 1994, vol. 653, 39-44
- **DELAHUNTY, T. M.** Mild traumatic brain injury enhances muscarinic receptor-linked inositol phosphate production in rat hippocampus. *Brain Res.,* 1992, vol. 594, 307-10
- **DELAHUNTY, T. M. ; JIANG, J. Y. ; BLACK, R. T. ; LYETH, B. G.** Differential modulation of carbachol and trans-ACPD-stimulated phosphoinositide turnover following traumatic brain injury. *Neurochem Res.,* 1995, vol. 20, 405-11
- **DELAHUNTY, T. M. ; JIANG, J. Y. ; GONG, Q. Z. ; BLACK, R. T. ; LYETH, B. G.** Differential consequences of lateral and central fluid percussion brain injury on receptor coupling in rat hippocampus. *J Neurotrauma.,* 1995, vol. 12, 1045-57

- **DHILLON, H. S. ; CARBARY, T. ; DOSE, J. ; DEMPSEY, R. J. ; PRASAD, M. R.** Activation of phosphatidylinositol bisphosphate signal transduction pathway after experimental brain injury: A lipid study. *Brain Research.,* 1995, vol. 698, 100-106
- **DIXON, C. E. ; LYETH, B. G. ; POVLISHOCK, J. T. ; FINDLING, R. L. ; HAMM, R. J. ; MARMAROU, A. ; YOUNG, H. F. ; HAYES, R. L.** A fluid percussion model of experimental brain injury in the rat. *J Neurosurg.,* 1987, vol. 67, 110-9
- **DIXON, C. E. ; MARKGRAF, C. G. ; ANGILERI, F. ; PIKE, B. R. ; WOLFSON, B. ; NEWCOMB, J. K. ; BISMAR, M. M. ; BLANCO, A. J. ; CLIFTON, G. L. ; HAYES, R. L.** Protective effects of moderate hypothermia on behavioral deficits but not necrotic cavitation following cortical impact injury in the rat. *Journal of Neurotrauma,* 1998, vol. 15, 95-103
- **DOODS, H. N. ; MATHY, M. J. ; DAVIDESKO, D. ; VAN CHARLDORP, K. J. ; DE JONGE, A. ; VAN ZWIETEN, P. A.** Selectivity of muscarinic antagonists in radioligand and in vivo experiments for the putative M1, M2 and M3 receptors. *Journal of Pharmacology and Experimental Therapeutics,* 1987, vol. 242, 257-262
- **FARKAS, O. ; LIFSHITZ, J. ; POVLISHOCK, J. T.** Mechanoporation induced by diffuse traumatic brain injury: an irreversible or reversible response to injury?. *J Neurosci.,* 2006, vol. 26, 3130-40
- **FINEMAN, I. ; HOVDA, D. A. ; SMITH, M. ; YOSHINO, A. ; BECKER, D. P.** Concussive brain injury is associated with a prolonged accumulation of calcium: a 45Ca autoradiographic study. *Brain Res.,* 1993, vol. 624, 94-102
- **FLEMINGER, S. ; OLIVER, D. L. ; LOVESTONE, S. ; RABE-HESKETH, S. ; GIORA, A.** Head injury as a risk factor for Alzheimer's disease: the evidence 10 years on; a partial replication. *J Neurol Neurosurg Psychiatry,* 2003, vol. 74, 857-62
- **GALLO, M. P. ; ALLOATTI, G. ; EVA, C. ; OBERTO, A. ; LEVI, R. C.** M1 muscarinic receptors increase calcium current and phosphoinositide turnover in guinea-pig ventricular cardiocytes. *J Physiol.,* 1993, vol. 471, 41-60
- **GORMAN, L. K. ; FU, K. ; HOVDA, D. A. ; BECKER, D. P. ; KATAYAMA, Y.** Analysis of acetylcholine release following concussive brain injury in the rat. *J Neurotrauma,* 1989, vol. 6, 203
- Acetylcholine metabolism in intracranial and lumbar cerebrospinal fluid and in blood. **HABER, B. ; GROSSMAN, R. G.** Neurobiology of Cerebrospinal Fluid. Plenum, 1980, vol. 1, 345-350
- **HALLAM, T. M. ; FLOYD, C. L. ; FOLKERTS, M. M. ; LEE, L. L. ; GONG, Q. Z. ; LYETH, B. G. ; MUIZELAAR, J. P. ; BERMNAN, R. F.** Comparison of behavioral deficits and acute neuronal degeneration in rat lateral fluid percussion and weight-drop brain injury models. *J Neurotrauma,* 2004, vol. 21, 521-39
- **HAMM, R. J. ; DIXON, C. E. ; GBADEBO, D. M. ; SINGHA, A. K. ; JENKINS, L. W. ; LYETH, B. G. ; HAYES, R. L.** Cognitive deficits following traumatic brain injury produced by controlled cortical impact. *J Neurotrauma,* 1992, vol. 9, 11-20
- **HEPPNER, F. ; DIEMATH, H. E.** Clinical experiences with anticholinergic treatment of closed intracranial trauma. *Monatsschrift fur Unfallheilkunde und Versicherungsmedizin,* 1958, vol. 61, 257-65
- **JENKNER, F. L. ; LECHNER, H.** The effects of scopolamine on the electroencephalogram in a new closed brain injury. *Der Nervenarzt,* 1954, vol. 25, 77-8
- **JENKNER, F. L. ; LECHNER, H.** The effect of diparcol on the electroencephalogram in the normal subject and in those with cerebral trauma. *Electroencephalogr Clin Neurophysiol.,* 1955, vol. 7, 303-5
- **JIANG, J. Y. ; LYETH, B. G. ; DELAHUNTY, T. M. ; PHILLIPS, L. L. ; HAMM, R. J.** Muscarinic cholinergic receptor binding in rat brain at 15 days following traumatic brain injury. *Brain Res.,* 1994, vol. 651, 123-8
- **JIANG, Z. W. ; GONG, Q. Z. ; DI, X. ; ZHU, J. ; LYETH, B. G.** Dicyclomine, an M1 muscarinic antagonist, reduces infarct volume in a rat subdural hematoma model. *Brain Res.,* 2000, vol. 852, 37-44
- **JOHNSON, G. V. ; JOPE, R. S. ; BINDER, L. I.** Proteolysis of tau by calpain. *Biochem Biophys Res Commun.,* 1989, vol. 163, 1505-11
- **KAMPFL, A. ; POSMANTUR, R. M. ; ZHAO, X. ; SCHMUTZHARD, E. ; CLIFTON, G. L. ; HAYES, R. L.** Mechanisms of calpain proteolysis following traumatic brain injury: Implications for pathology and therapy: A review and update. *J.Neurotrauma,* 1997, vol. 14, 121-134
- **KUMAGAE, Y. ; MATSUI, Y.** Output, tissue levels, and synthesis of acetylcholine during and after transient forebrain ischemia in the rat. *Journal of Neurochemistry,* 1991, vol. 56, 1169-1173
- **LANGLOIS, J. A. ; RUTLAND-BROWN, W. ; THOMAS, K. E.** Traumatic Brain Injury in the United States: Emergency Department Visits, Hospitalization, and Deaths, vol. Atlanta (GA) Centers for Disease Control and Prevention. National Center for Injury Prevention and Control, 2006
- **LEWIS, S. B. ; VELAT, G. J. ; MIRALIA, L. ; PAPA, L. ; AIKMAN, J. M. ; WOLPER, R. A. ; FIRMENT, C. S. ; LIU, M. C. ; PINEDA, J. A. ; WANG, K. K.** Alpha-II spectrin breakdown products in aneurysmal subarachnoid hemorrhage: a novel biomarker of proteolytic injury. *J Neurosurg.,* 2007, vol. 107, 792-6
- **LIU, X. ; VAN VLEET, T. ; SCHNELLMANN, R. G.** The role of calpain in oncotic cell death. *Annu Rev Pharmacol Toxicol.,* 2004, vol. 44, 349-70

- **LYE, T. C. ; SHORES, E. A.** Traumatic brain injury as a risk factor for Alzheimer's disease: a review. *Neuropsychology review,* 2000, vol. 10, 115-29
- **LYETH, B. G. ; DIXON, C. E. ; HAMM, R. J. ; JENKINS, L. W. ; YOUNG, H. F. ; STONNINGTON, H. H. ; HAYES, R. L.** Effects of anticholinergic treatment on transient behavioral suppression and physiological responses following concussive brain injury to the rat. *Brain Res.,* 1988, vol. 448, 88-97
- **LYETH, B. G. ; DIXON, C. E. ; JENKINS, L. W. ; HAMM, R. J. ; ALBERICO, A. ; YOUNG, H. F. ; STONNINGTON, H. H. ; HAYES, R. L.** Effects of scopolamine treatment on long-term behavioral deficits following concussive brain injury to the rat. *Brain Res.,* 1988, vol. 452, 39-48
- **LYETH, B. G. ; GONG, Q. Z. ; DHILLON, H. S. ; PRASAD, M. R.** Effects of muscarinic receptor antagonism on the phosphatidylinositol bisphosphate signal transduction pathway after experimental brain injury. *Brain Res.,* 1996, vol. 742, 63-70
- **LYETH, B. G. ; HAYES, R. L.** Cholinergic and opioid mediation of traumatic brain injury. *J Neurotrauma,* 1992, vol. 9 (2), 463-74
- **LYETH, B. G. ; JENKINS, L. W. ; HAMM, R. J. ; DIXON, C. E. ; PHILLIPS, L. L. ; CLIFTON, G. L. ; YOUNG, H. F. ; HAYES, R. L.** Prolonged memory impairment in the absence of hippocampal cell death following traumatic brain injury in the rat. *Brain Res.,* 1990, vol. 526, 249-58
- **LYETH, B. G. ; JIANG, J. Y. ; DELAHUNTY, T. M. ; PHILLIPS, L. L. ; HAMM, R. J.** Muscarinic cholinergic receptor binding in rat brain following traumatic brain injury. *Brain Res.,* 1994, vol. 640, 240-5
- **LYETH, B. G. ; JIANG, J. Y. ; ROBINSON, S. E. ; GUO, H. ; JENKINS, L. W.** Hypothermia blunts acetylcholine increase in CSF of traumatically brain injured rats. *Mol Chem Neuropathol.,* 1993, vol. 18, 247-56
- **LYETH, B. G. ; LIU, S. ; HAMM, R. J.** Combined scopolamine and morphine treatment of traumatic brain injury in the rat. *Brain Res.,* 1993, vol. 617, 69-75
- **MANDELKOW, E. M. ; MANDELKOW, E.** Tau in Alzheimer's disease. *Trends in cell biology,* 1998, vol. 8, 425-7
- **MAX, W. ; MACKENZIE, E. J. ; RICE, D. P.** Head injuries: costs and consequences. *J Head Trauma Rehabil,* 1991, vol. 6, 76-91
- **MCALLISTER, T. W.** Neuropsychiatric sequelae of head injuries. *The Psychiatric clinics of North America,* 1992, vol. 15, 395-413
- **MCINTOSH, T. K. ; VINK, R. ; NOBLE, L. ; YAMAKAMI, I. ; FERNYAK, S. ; SOARES, H. ; FADEN, A. I.** Traumatic brain injury in the rat: characterization of a lateral fluid-percussion model. *Neuroscience,* 1989, vol. 28, 233-244

- **MIYAZAKI, S. ; KATAYAMA, Y. ; LYETH, B. G. ; JENKINS, L. W. ; DEWITT, D. S. ; GOLDBERG, S. J. ; NEWLON, P. G. ; HAYES, R. L.** Enduring suppression of hippocampal long-term potentiation following traumatic brain injury in rat. *Brain Res.,* 1992, vol. 585, 335-9
- **OLNEY, J. W. ; DE GUBAREFF, T. ; LABRUYERE, J.** Seizure-related brain damage induced by cholinergic agents. *Nature,* 1983, vol. 301, 520-522
- **PIKE, B. R. ; FLINT, J. ; DAVE, J. R. ; LU, X. C. ; WANG, K. K. ; TORTELLA, F. C. ; HAYES, R. L.** Accumulation of calpain and caspase-3 proteolytic fragments of brain-derived alphaII-spectrin in cerebral spinal fluid after middle cerebral artery occlusion in rats. *J Cereb Blood Flow Metab,* 2004, vol. 24, 98-106
- **PIKE, B. R. ; FLINT, J. ; DUTTA, S. ; JOHNSON, E. ; WANG, K. K. ; HAYES, R. L.** Accumulation of non-erythroid alpha II-spectrin and calpain-cleaved alpha II-spectrin breakdown products in cerebrospinal fluid after traumatic brain injury in rats. *J Neurochem.,* 2001, vol. 78, 1297-306
- **PINEDA, J. A. ; LEWIS, S. B. ; VALADKA, A. B. ; PAPA, L. ; HANNAY, H. J. ; HEATON, S. C. ; DEMERY, J. A. ; LIU, M. C. ; AIKMAN, J. M. ; AKLE, V.** Clinical significance of alphaII-spectrin breakdown products in cerebrospinal fluid after severe traumatic brain injury. *J Neurotrauma,* 2007, vol. 24, 354-66
- **PRASAD, M. R. ; DHILLON, H. S. ; CARBARY, T. ; DEMPSEY, R. J. ; SCHEFF, S. W.** Enhanced phosphodiestric breakdown of phosphatidylinositol bisphosphate after experimental brain injury. *J Neurochem.,* 1994, vol. 63, 773-776
- **REEVES, T. M. ; LYETH, B. G. ; POVLISHOCK, J. T.** Long-term potentiation deficits and excitability changes following traumatic brain injury. *Exp Brain Res.,* 1995, vol. 106, 248-56
- **RIEDERER, B. M. ; ZAGON, I. S. ; GOODMAN, S. R.** Brain spectrin(240/235) and brain spectrin(240/235E): two distinct spectrin subtypes with different locations within mammalian neural cells. *J Cell Biol.,* 1986, vol. 102, 2088-97
- **RINGGER, N. C. ; O'STEEN, B. E. ; BRABHAM, J. G. ; SILVER, X. ; PINEDA, J. ; WANG, K. K. ; HAYES, R. L. ; PAPA, L.** A novel marker for traumatic brain injury: CSF alphaII-spectrin breakdown product levels. *J Neurotrauma,* 2004, vol. 21, 1443-56
- **ROBINSON, S. E. ; FOXX, S. D. ; POSNER, M. G. ; MARTIN, R. M. ; DAVIS, T. R. ; GUO, H. ; ENTERS, E. K.** The effect of M1 muscarinic blockade on behavior and physiological responses following traumatic brain injury in the rat. *Brain Res.,* 1990, vol. 511, 141-148
- **SACHS, E., JR.** Acetylcholine and serotonin in the spinal fluid. *J Neurosurg.,* 1957, vol. 14, 22-7

- **SAIDO, T. C. ; SORIMACHI, H. ; SUZUKI, K.** Calpain: new perspectives in molecular diversity and physiological-pathological involvement. *Faseb J.,* 1994, vol. 8, 814-22
- **SCHMUED, L. C. ; ALBERTSON, C. ; SLIKKER, W. J.** Fluoro-Jade: a novel fluorochrome for the sensitive and reliable histochemical localization of neuronal degeneration. *Brain Research.,* 1997, vol. 751, 37-46
- **SCHMUED, L. C. ; HOPKINS, K. J.** Fluoro-Jade: novel fluorochromes for detecting toxicant-induced neuronal degeneration. *Toxicologic Pathology,* 2000, vol. 28, 91-99
- **SHARMA, V. K. ; COLECRAFT, H. M. ; WANG, D. X. ; LEVEY, A. I. ; GRIGORENKO, E. V. ; YEH, H. H. ; SHEU, S. S.** Molecular and functional identification of m1 muscarinic acetylcholine receptors in rat ventricular myocytes. *Circ Res.,* 1996, vol. 79, 86-93
- **THOMPSON, H. J. ; LIFSHITZ, J. ; MARKLUND, N. ; GRADY, M. S. ; GRAHAM, D. I. ; HOVDA, D. A. ; MCINTOSH, T. K.** Lateral fluid percussion brain injury: a 15-year review and evaluation. *J Neurotrauma,* 2005, vol. 22, 42-75
- **TOWER, D. B. ; MCEACHERN, D.** Acetylcholine and neuronal activity; cholinesterase patterns and acetylcholine in the cerebrospinal fluids of patients with craniocerebral trauma. *Canadian journal of research,* 1949, vol. 27, 105-19
- **TURSKI, W. A. ; CZUCZWAR, S. J. ; KLEINROK, Z. ; TURSKI, L.** Cholinomimetics produce seizures and brain damage in rats. *Experientia,* 1983, vol. 39, 1408-11
- **WANG, K. K.** Calpain and caspase: can you tell the difference?. *Trends Neurosci.,* 2000, vol. 23, 20-6
- **WANG, K. K. ; OTTENS, A. K. ; LIU, M. C. ; LEWIS, S. B. ; MEEGAN, C. ; OLI, M. W. ; TORTELLA, F. C. ; HAYES, R. L.** Proteomic identification of biomarkers of traumatic brain injury. *Expert review of proteomics,* 2005, vol. 2, 603-14
- Calpain and caspase in ischemic and traumtic brain injury. **WANG, K. K. W.** New Concepts in Cerebral Ischemia. CRC Press, 2002, 181-200
- **WARD JR., A.** Atropine in the treatment of closed head injury. *J Neurosurg.,* 1950, vol. 7, 398-402
- **WEI, E. P. ; LAMB, R. G. ; KONTOS, H. A.** Increased phospholipase C activity after experimental brain injury. *J Neurosurg.,* 1982, vol. 56, 695-8
- **WEST, M. J. ; SLOMIANKA, L. ; GUNDERSEN, H. J.** Unbiased stereological estimation of the total number of neurons in the subdivisions of the rat hippocampus using the optical fractionator. *Anatomical Record.,* 1991, vol. 231, 482-497
- **BERGER RP ; ADELSON PD ; PIERCE MC ; DULANI T ; CASSIDY LD ; KOCHANEK PM.** Serum neuron-specific enolase, S100B, and myelin basic protein concentrations after inflicted and noninflicted traumatic brain injury in children. *J Neurosurg,* 2005, vol. 103, 61-68
- **CHOI DW.** Exploratory clinical testing of neuroscience drugs. *Nat Neurosci,* 2002, 1023-1025
- **CHOI SC ; BULLOCK R.** Design and statistical issues in multicenter trials of severe head injury. *Neurol Res,* 2001, vol. 23, 190-192
- **COLEMAN MP ; RIBCHESTER RR.** Programmed axon death, synaptic dysfunction and the ubiquitin proteasome system. *Curr Drug Targets CNS Neurol Disord,* 2004, vol. 3, 227-238
- **COOPER EH.** Neuron-specific enolase. *Int J Biol Markers,* 1994, vol. 9, 205-210
- **DAMBINOVA SA ; KHOUNTEEV GA ; IZYKENOVA GA ; ZAVOLOKOV IG ; ILYUKHINA AY ; SKOROMETS AA.** Blood test detecting autoantibodies to N-methyl-D-aspartate neuroreceptors for evaluation of patients with transient ischemic attack and stroke. *Clin Chem,* 2003, vol. 49, 1752-1762
- **DORAN JF ; JACKSON P ; KYNOCH PA ; THOMPSON RJ.** Isolation of PGP 9.5, a new human neurone-specific protein detected by high-resolution two-dimensional electrophoresis. *J Neurochem,* 1983, vol. 40, 1542-1547
- **GE P ; LUO Y ; LIU CL ; HU B.** Protein aggregation and proteasome dysfunction after brain ischemia. *Stroke,* 2007, vol. 38, 3230-3236
- **GONG B ; LEZNIK E.** The role of ubiquitin C-terminal hydrolase L1 in neurodegenerative disorders. *J Cereb Blood Flow Metab,* 2007, vol. 20, 365-370
- **HU BR ; JANELIDZE S ; GINSBERG MD ; BUSTO R ; PEREZ-PINZON M ; SICK TJ ; SIESJO BK ; LIU CL.** Protein aggregation after focal brain ischemia and reperfusion. *J Cereb Blood Flow Metab,* 2001, vol. 21, 865-875
- **JACKSON P ; THOMPSON RJ.** The demonstration of new human brain-specific proteins by high-resolution two-dimensional polyacrylamide gel electrophoresis. *J Neurol Sci,* 1981, vol. 49, 429-438
- **JOHNSSON P ; BLOMQUIST S ; LÜHRS C ; MALMKVIST G ; ALLING C ; SOLEM JO ; STAHL E.** Neuron-specific enolase increases in plasma during and immediately after extracorporeal circulation. *Ann Thorac Surg,* 2000, vol. 69, 750-4
- **JOHNSTON SC ; RIDDLE SM ; COHEN RE ; HILL CP.** Structural basis for the specificity of ubiquitin C-terminal hydrolases. *EMBO J,* 1999, vol. 18, 3877-3887
- **KELLER JN ; HUANG FF ; ZHU H ; YU J ; HO YS ; KINDY TS.** Oxidative stress-associated impairment of proteasome activity during ischemia-reperfusion injury. *J Cereb Blood Flow Metab,* 2000, vol. 20, 1467-1473

- **KOBEISSY FH ; OTTENS AK ; ZHANG ZQ ; DAVE JR ; TORTELLA FC ; HAYES RL ; WANG KKW.** Novel differential neuroproteomics analysis of traumatic brain injury in rats. *Mol Cell Proteomics,* 2006, vol. 5, 1887-1898
- **KWON J ; MOCHIDA K ; WANG YL ; SEKIGUCHI S ; SANKAI T ; AOKI S ; OGURA A ; YOSHIKAWA Y ; WADA K.** Ubiquitin C-terminal hydrolase L-1 is essential for the early apoptotic wave of germinal cells and for sperm quality control during spermatogenesis. *Biol Reprod,* 2005, vol. 73, 29-35
- **LANGLOIS JA ; RUTLAND-BROWN W ; THOMAS KE.** Traumatic brain injury in the United States: Emergency department visits, hospitalizations, and deaths. *National Center for Injury Prevention and Control, Centers for Disease Control and Prevention,* 2004
- **LARSEN CN ; PRICE JS ; WILKINSON KD.** Substrate binding and catalysis by ubiquitin C-terminal hydrolases: identification of two active site residues. *Biochemistry,* 1996, vol. 35, 6735-6744
- **LARSEN CN ; KRANTZ BA ; WILKINSON KD.** Substrate specificity of deubiquitinating enzymes: ubiquitin C-terminal hydrolases. *Biochemistry,* 1998, vol. 37, 3358-3368
- **LASER H ; MACK TG ; WAGNER D ; COLEMAN MP.** Proteasome inhibition arrests neurite outgrowth and causes ''dying-back'' degeneration in primary culture. *J Neurosci Res,* 2003, vol. 74, 906-916
- **LINCOLN S ; VAUGHAN J ; WOOD N ; BAKER M ; ADAMSON J ; GWINN-HARDY K ; LYNCH T ; HARDY J ; FARRER M.** Low frequency of pathogenic mutations in the ubiquitin carboxy-terminal hydrolase gene in familial Parkinson's disease. *Neuroreport,* 1999, vol. 10, 427-429
- **MISSLER U ; WIESMANN M ; FRIEDRICH C ; KAPS M.** S-100 protein and neuron-specific enolase concentrations in blood as indicators of infarction volume and prognosis in acute ischemic stroke. *Stroke,* 1997, vol. 28, 1956-1960
- **PELINKA LE ; SZALAY L ; JAFARMADAR M ; SCHMIDHAMMER R ; REDL H ; BAHRAMI S.** Circulating S100B is increased after bilateral femur fracture without brain injury in the rat. *Br J Anaesth.,* 2003, vol. 91, 595-7
- **PELINKA LE ; KROEPFL A ; SCHMIDHAMMER R ; KRENN M ; BUCHINGER W ; REDL H ; RAABE A.** Glial fibrillary acidic protein in serum after traumatic brain injury and multiple trauma. *J Trauma,* 2004, vol. 57, 1006-1012
- **PELINKA LE ; KROEPFL A ; LEIXNERING M ; BUCHINGER W ; RAABE A ; REDL H.** GFAP versus S100B in serum after traumatic brain injury: relationship to brain damage and outcome. *J Neurotrauma,* 2004, vol. 21, 1553-1561
- **PELINKA LE ; HARADA N ; SZALAY L ; JAFARMADAR M ; REDL H ; BAHRAMI S.** Release of S100B differs during ischemia and reperfusion of the liver, the gut, and the kidney in rats. *Shock,* 2004, vol. 21, 72-6
- **PIKE BR ; FLINT J ; DAVE JR ; LU XC ; WANG KKW ; TORTELLA FC ; HAYES RL.** Accumulation of calpain and caspase-3 proteolytic fragments of brain-derived αII-spectrin in CSF after middle cerebral artery occlusion in rats. *J Cereb Blood Flow Metab,* 2003, vol. 24, 98-106
- **PIKE BR ; FLINT J ; JOHNSON E ; GLENN CC ; DUTTA S ; WANG KKW ; HAYES RL.** Accumulation of Calpain-Cleaved Non-Erythroid αII-Spectrin in Cerebrospinal Fluid after Traumatic Brain Injury in Rats. *J Neurochem,* 2001, vol. 78, 1297-1306
- **RAABE A ; GROLMS C ; SEIFERT V.** Serum markers of brain damage and outcome prediction in patients after severe head injury. *Br J Neurosurg,* 1999, vol. 13, 56-59
- **ROMNER B ; INGEBRIGTSEN T ; KONGSTAD P ; BORGESEN SE.** Traumatic brain damage: serum S-100 protein measurements related to neuroradiological findings. *J Neurotrauma,* 2000, vol. 17, 641-647
- **ROSS SA ; CUNNINGHAM RT ; JOHNSTON CF ; ROWLANDS BJ.** Neuron-specific enolase as an aid to outcome prediction in head injury. *Br J Neurosurg,* 1996, vol. 10, 471-476
- **SAIGOH K ; WANG YL ; SUH JG ; YAMANISHI T ; SAKAI Y ; KIYOSAWA H ; HARADA T ; ICHIHARA N ; WAKANA S ; KIKUCHI T.** Intragenic deletion in the gene encoding ubiquitin carboxy-terminal hydrolase in gad mice. *Nat Genet,* 1999, vol. 23, 47-51
- **SHAW GJ ; JAUCH EC ; ZEMLAN FP.** Serum cleaved tau protein levels and clinical outcome in adult patients with closed head injury. *Ann Emerg Med,* 2002, vol. 39, 254-257
- **THOMPSON RJ ; DORAN JF ; JACKSON P ; DHILLON AP ; RODE J.** PGP 9.5--a new marker for vertebrate neurons and neuroendocrine cells. *Brain Res,* 1983, vol. 278, 224-228
- **TONGAONKAR P ; CHEN L ; LAMBERTSON D ; KO B ; MADURA K.** Evidence for an interaction between ubiquitin-conjugating enzymes and the 26S proteasome. *Mol Cell Biol,* 2000, vol. 20, 4691-4698
- **VOS PE ; LAMERS KJ ; HENDRIKS JC ; VAN HAAREN M ; BEEMS T ; ZIMMERMAN C ; VAN GEEL W ; DE REUS H ; BIERT J ; VERBEEK MM.** Glial and neuronal proteins in serum predict outcome after severe traumatic brain injury. *Neurology,* 2004, vol. 62, 1303-1310

- **WANG KKW ; OTTENS A ; LIU MC ; LEWIS SB ; MEEGAN C ; OLI MW ; TORTELLA FC ; HAYES RL.** Proteomic identification of biomarkers of traumatic brain injury. *Expert Reviews Proteomics,* 2005, vol. 2, 603-614
- **WILKINSON KD ; DESHPANDE S ; LARSEN CN.** Comparisons of neuronal (PGP9.5) and non-neuronal ubiquitin C-terminal hydrolases. *Biochem Soc Trans,* 1992, vol. 20, 631-637
- **WILKINSON KD.** Roles of ubiquitinylation in proteolysis and cellular regulation. *Ann Rev Nutr,* 1995, vol. 15, 161-189
- **WILKINSON KD.** Regulation of ubiquitin-dependent processes by deubiquitinating enzymes. *FASEB J,* 1997, vol. 11, 1245-1256
- **YAMAZAKI Y ; YADA K ; MORII S ; KITAHARA T ; OHWADA T.** Diagnostic significance of serum neuron-specific enolase and myelin basic protein assay in patients with acute head injury. *Surg Neurol,* 1995, vol. 43, 267-270
- **ZEMLAN FP ; JAUCH EC ; MULCHAHEY JJ ; GABBITA SP ; ROSENBERG WS ; SPECIALE SG ; ZUCCARELLO M.** C-tau biomarker of neuronal damage in severe brain injured patients: association with elevated intracranial pressure and clinical outcome. *Brain Res,* 2002, vol. 947, 131-139